# EUROPEAN PATENT SPECIFICATION

(11) **EP 4 347 753 B1**
(45) Date of publication and mention of the grant of the patent: **19.11.2025**
(21) Application number: 22810778.5
(22) Date of filing: 30.05.2022
(51) Int. Cl.: C10M 133/06, C10M 135/18, C10M 125/22, C10M 159/18, C10M 169/02, C10N 30/06, C10M 125/24, C10M 137/14, C10N 20/00, C10N 30/02, C07D 401/12, C07F 11/00, C07D 233/58, C10N 30/12, C10N 40/25, C10N 50/10, C10N 70/00

(54) **FRICTION AND WEAR REDUCTION ADDITIVES**
REIBUNGS- UND VERSCHLEISSMINDERUNGSADDITIVE
ADDITIFS DE RÉDUCTION DE FROTTEMENT ET D'USURE

(30) Priority: 28.05.2021 MY PI2021002997
(43) Date of publication of application: 10.04.2024
(73) Proprietor: Petroliam Nasional Berhad (Petronas), Kuala Lumpur 50088 (MY)
(72) Inventor: PEROTTO, Carlo, Kuala Lumpur, 50088 (MY); YASIN, Farah Fazlina M., Kuala Lumpur, 50088 (MY); BELLINO, Francesco, Kuala Lumpur, 50088 (MY); DOLFI, Andrea, Kuala Lumpur, 50088 (MY); SRINIVASAN, Geetha, Kuala Lumpur, 50088 (MY)
(74) Representative: Hamer, Christopher K.
(86) International application number: PCT/IB2022/055065
(87) International publication number: WO 2022/249157

(56) References cited:
- WO-A1-2021/005372
- US-A1- 2017 240 838
- US-A1- 2017 296 553
- PARENAGO O. P.; KUZ’MINA G. N.; ZAIMOVSKAYA T. A.: "Sulfur-containing molybdenum compounds as high-performance lubricant additives (Review)", PETROLEUM CHEMISTRY, PLEIADES PUBLISHING, MOSCOW, vol. 57, no. 8, 22 July 2017 (2017-07-22), Moscow, pages 631 - 642, XP036282150, ISSN: 0965-5441, DOI: 10.1134/S0965544117080102
- MÜLLER A., E. KRICKEMEYER, UTA REINSCH: "Reaktionen von koordinierten S22--liganden.I reaktion von [Mo2(S2)6]2- mit nucleophilen und einfache darstellung des bis-disulfido-komplexes [Mo2O2S2(S2)2]2-", ZEITSCHRIFT FÜR ANORGANISCHE UND ALLGEMEINE CHEMIE, vol. 470, no. 1, 30 November 1980 (1980-11-30), pages 35 - 38, XP093008554, DOI: 10.1002/zaac.19804700105
- KIW YU MIN, SCHAEFFER PHILIPPE, ADAM PIERRE, THIÉBAUT BENOÎT, BOYER CHANTAL, PAPIN GÉRALDINE: "Ligand exchange processes between molybdenum and zinc additives in lubricants: evidence from NMR ( 1 H, 13 C, 31 P) and HPLC-MS analysis", RSC ADVANCES, vol. 10, no. 62, 14 October 2020 (2020-10-14), pages 37962 - 37973, XP093008556, DOI: 10.1039/D0RA07329F

## Description

### FIELD OF THE INVENTION

The present invention relates to additives for reducing friction and wear in lubricant compositions such as lubricating oils and greases. In particular, the present invention relates to novel salts and their use in lubricating oil compositions and grease compositions for reducing friction and wear.

### BACKGROUND OF THE INVENTION

Lubricant compositions generally comprise a base oil of lubricating viscosity together with one or more additives to deliver properties such as reduced friction and wear, improved viscosity index, detergency, and resistance to oxidation and corrosion. A lubricant base oil may comprise one or more sources of lubricating oil, referred to as base stocks. Typically, lubricants contain about 90% by weight of base oil and around 10% by weight of additives. Lubricant base stocks may be derived from crude oil. Such base stocks are known as mineral oils. Such lubricant base stocks useful in automotive engine lubricants may be obtained as higher boiling fractions from the refining of crude oil or via synthetic routes, and are classified as Group I, II, III, IV and V base stocks according to API standard 1509, "ENGINE OIL LICENSING AND CERTIFICATION SYSTEM", April 2007 version 16th edition Appendix E. Lubricant base stocks may also be obtained synthetically using synthetic hydrocarbons (that may themselves be derived from petroleum). Such base stocks are known as synthetic base stocks and include polyalpha-olefins (PAO), synthetic esters, polyalkylene glycols (PAG), phosphate esters, alkylated naphthalenes (AN), silicate esters, ionic fluids and multiply alkylated cyclopentanes (MAC).

Lubricating oil compositions have a variety of uses. A principal use of said compositions is in lubricating the moving parts of internal combustion engines in motor vehicles and powered equipment such as spark ignition engines and compression ignition engines. Lubricating oil compositions generally comprise a variety of additives to aid the lubricating oils in performing their functions. Friction reduction additives are generally included in lubricating oil compositions to reduce the friction between moving parts of the system. Anti-wear additives are also typically included to reduce the wear upon the surfaces of the moving parts. An example of commercially available friction reduction additive is the compound known as molybdenum dithiocarbamate MoDTC which has the formula:

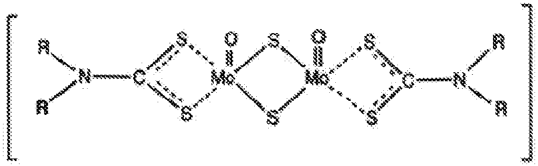

An example of a commercially available anti-wear additive used in lubricating oil compositions are the compounds known as zinc dialkyl and diaryldithiophosphates (ZDDPs) which have the following formula:

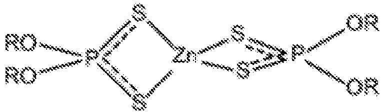

Grease compositions are solid or semisolid lubricant compositions formed as a dispersion of thickening agents in a liquid lubricant, such as the lubricating oils discussed above. Typically, grease compositions comprise lubricating oil in an amount of from 50% to 95% by weight of the grease composition and more typically in an amount of 70% to 95% by weight of the composition. Grease compositions typically containing thickeners in an amount of from 5% to 50% by weight of the composition and more typically in an amount of from 5% to 30% by weight of the composition. Typical thickeners include soaps such as fatty acid salts of alkali and alkaline earth metals. Greases typically possess high initial viscosity which drops upon the application of shear. Typically, shear forces cause the grease viscosity to reduce to thatof the base oil used in the grease. Greases are particularly useful as lubricants in applications where a lubricating oil would not stay in position or where a mechanism can only have lubricant applied infrequently. Greases desirably comprise anti-friction and anti-wear additives such as those discussed above for the same purposes as lubricating oil compositions.

Various salts are also known to be used in lubricating oil compositions to provide friction reduction and anti-wear properties to the composition. WO2008/075016 discloses various salts for use in lubricating oil compositions to provide anti-wear and friction reduction properties. The salts comprise quaternary ammonium and quaternary phosphonium cations having four hydrocarbyl groups. The salts comprise anions of the formula [R¹R²P(O)O]⁻, carboxylate anions and various sulfosuccinate ester anions.

Further salts for use in lubricating oil compositions as friction reduction and anti-wear additives are disclosed in US2017/0240838 and US2017/0240837 and have the following formula:

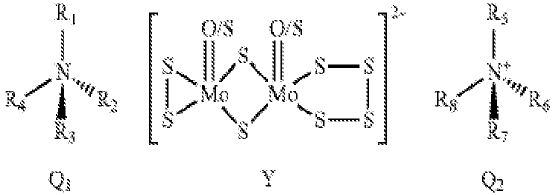

The anions of the salts disclosed in said documents are limited to asymmetric binuclear anions of the above formula. Furthermore, the cations of the salts disclosed in said documents are limited to imidazolium cations and quaternary ammonium cations substituted with hydrocarbyl groups.

US4370245 discloses compounds with the following formula:

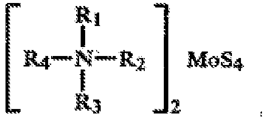

Groups R₁ to R₃ are limited to alkyl and alkenyl groups having from 1 to 30 carbon atoms and R₄ is limited to alkyl and alkenyl groups having from 4 to 30 carbon atoms. The salts are disclosed as improving the extreme pressure properties of greases comprising substituted urea thickeners. It is taught that said salts do not improve the properties of other greases such as those thickened with alkali metals or alkali earth metals. Furthermore, it is taught that different hydrocarbylammonium thiomolybdates to those disclosed in the document are not effective with the substituted-urea thickened greases at improving their extreme pressure properties.

US4400282 discloses lubricating oil compositions comprising certain tetrahydrocarbyl ammonium thiomolybdates where the anion has the formula MoS₄²⁻. The thiomolybdate salts are included in the compositions as anti-friction additives.

WO2021/005372 discloses lubricating oil compositions comprising additives that impart anti-friction and anti-wear properties to the lubricating oil compositions. The additives are ionic liquids comprising various cations such as certain phosphonium, ammonium and sulfonium cations and Group 6 metal mononuclear metallate anions such as MoS₄²⁻. The ionic liquids of the disclosure are limited to mononuclear species.

The inventors of the present invention have appreciated that there is a continued need for new and improved additives for use in lubricant compositions that improve the anti-wear properties of said compositions and that reduce friction in said compositions. The inventors have further appreciated that whilst the ionic liquids based on mononuclear metallate anions of WO2021/005372 impart useful anti-wear and anti-friction properties to the lubricating oil compositions in which they are present, said ionic liquids often have limited solubility and stability when present in certain lubricant base oils such as engine oil with industrial standard lubricant packages. The limited stability means that the ionic liquids may break down into species with reduced anti-friction and anti-wear properties when present in the lubricating oil negatively affecting the properties of the oil. The limited solubility may mean that less ionic liquid is effectively dissolved within the lubricating oil and able to impart anti-friction and anti-wear properties to the oil. The inventors have thus appreciated that there is a need for anti-friction and anti-wear additives for use in lubricant compositions that are as good or better at imparting anti-friction and anti-wear properties to the lubricant compositions as the ionic liquids disclosed in WO2021/005372 but that have improved stability and solubility when present in lubricating oil and grease compositions.

### SUMMARY OF THE INVENTION

The present invention is based, in part, on the surprising finding that certain novel ionic liquid salts impart improved anti-wear properties and/or improved friction reduction properties to lubricant compositions such as lubricating oil compositions and grease compositions. In particular, it has been found that certain novel ionic liquid salts impart improved or as good anti-wear and friction reduction properties to lubricant compositions such as lubricating oil compositions and grease compositions as the ionic liquids disclosed in WO2021/005372, whilst providing improved solubility and stability when present in lubricant compositions when compared to the ionic liquids disclosed in WO2021/005372.

According to a first aspect of the invention, there is provided a lubricant composition comprising one or more base oils and one or more ionic liquids, wherein the one or more ionic liquids comprise one or more cations and one or more binuclear molybdenum-containing metallate anions, wherein the one or more binuclear molybdenum-containing metallate anions is of the formula [Mo₂A₃C_{d}] and has a charge of -1 or -2, wherein A and C are each independently selected from O and S; and wherein b and d are each from 0 to 8; wherein the sum of b and d is 8.

The term "ionic liquid" as used herein refers to a liquid that is capable of being produced by melting a salt, and when so produced consists solely of ions. An ionic liquid may be formed from a homogeneous substance comprising one species of cation and one species of anion, or it can be composed of more than one species of cation and/or more than one species of anion. Thus, an ionic liquid may be composed of more than one species of cation and one species of anion. An ionic liquid may further be composed of one species of cation, and one or more species of anion. Still further, an ionic liquid may be composed of more than one species of cation and more than one species of anion.

The term "ionic liquid" includes compounds having both high melting points and compounds having low melting points, e.g. at or below room temperature. Thus, many ionic liquids have melting points below 200 °C, preferably below 150 °C, particularly below 100 °C, around room temperature (15 to 30 °C), or even below 0 °C. Ionic liquids having melting points below around 30°C are commonly referred to as "room temperature ionic liquids". In room temperature ionic liquids, the structures of the cation and anion prevent the formation of an ordered crystalline structure and therefore the salt is liquid at room temperature.

Ionic liquids are most widely known as solvents, because of their negligible vapour pressure, temperature stability, low flammability and recyclability make them environmentally friendly. Due to the vast number of anion/cation combinations that are available it is possible to fine-tune the physical properties of the ionic liquid (e.g. melting point, density, viscosity, and miscibility with water or organic solvents) to suit the requirements of a particular application.

The term binuclear as used herein is used to refer to anions that contain two central metal atoms with one or more ligands bonded thereto. This is in contrast to mononuclear anions that contain one central metal atom with one or more ligands bonded thereto, or polynuclear anionic complexes that comprise more than two central metal atoms with ligands bonded thereto. In the binuclear anions present in ionic liquids of the present disclosure, the binuclear complexes typically contain one or more ligands bonded to both central metal atoms present in the binuclear anions. Such ligands may be referred to as "bridging ligands" between the metal centres. Typically, the binuclear anions present in the ionic liquids of the present disclosure contain two bridging ligands, each bonded to both central metal atoms of the anion.

The one or more anions of the ionic liquids of the present disclosure are binuclear and comprise two molybdenum atoms.

The anion may have any possible coordination geometry of the ligands around the central molybdenum atoms. Preferably, the central molybdenum atoms of the anion each have five atoms ligated thereto, although it will be understood by the skilled person that the anion may have different coordination geometries with different numbers of ligating atoms bonded to the central molybdenum atoms.

The one or more anions of the ionic liquids of the present disclosure comprise at least one oxygen atom or at least one sulphur atom. Preferably, the one or more anions comprise at least one sulphur atom and at least one oxygen atom. The sum of the number of oxygen atoms and sulphur atoms in the one or more anions of the ionic liquids of the present disclosure is eight. Thus, the one or more anions may comprise eight sulphur atoms and no oxygen atoms; eight oxygen atoms and no sulphur atoms; or a mixture of oxygen and sulphur atoms provided the sum of the number of oxygen atoms and sulphur atoms is eight. The sulphur atoms, if present, may be singly or doubly bonded to one or both of the molybdenum atoms present. Similarly, the oxygen atoms, if present, may be singly or doubly bonded to one or both of the molybdenum atoms present.

Preferably, all oxygen atoms in the one or more anions are double bonded to molybdenum and all sulphur atoms present are singly bonded to molybdenum.

Preferably, the one or more anions comprise two bridging ligands which are each singly bonded to both molybdenum atoms present. More preferably, both bridging ligands are sulphur atoms.

Preferably, the one or more binuclear molybdenum-containing metallate anions has a charge of - 2.

Preferably, each molybdenum atom is ligated to 5 atoms.

Typically, the one or more binuclear molybdenum-containing metallate anions is of the formula: 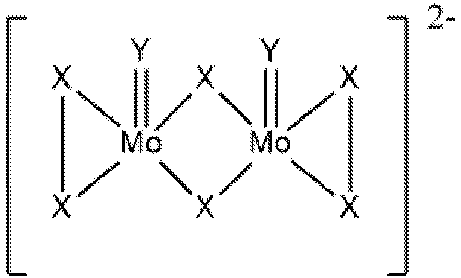 wherein each X and each Y is independently selected from O or S. Preferably, the one or more binuclear molybdenum-containing metallate anions comprise at least two O and/or at least two S atoms. More preferably, the one or more binuclear molybdenum-containing metallate anions comprise at least two O and at least two S atoms.

In some instances, where the one or more anions have the formula depicted above, at least one X is O and at least one Y is S.

In other, more preferable, instances, where the one or more anions have the formula depicted above, at least one X is S and at least one Y is O. More preferably, each X is S and at least one Y is O. Most preferably, each X is S and each Y is O.

In other instances, the anions of the disclosure have the structural formula depicted above where each X is S and each Y is S. In this instance, the anion has the formula [Mo₂S₈]²⁻. In highly preferable instances, the one or more ionic liquids have the formula: 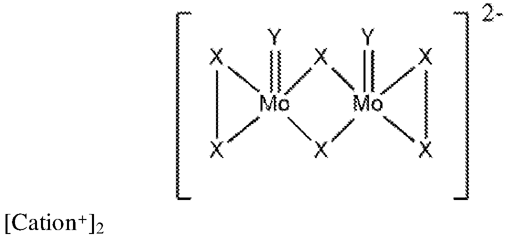 wherein X and Y are as defined above and wherein the cation is as described in further detail below. Most preferably, each X is S and each Y is O.

In some instances, ionic liquids of the present disclosure comprise anions that may have one or more of the above structural formulae. In some instances, the anions may exist in a state of equilibrium between the structures described above.

The ionic liquids of the present disclosure comprise one or more cations.

Typically, the one or more cations comprise one or more cations selected from an imidazolium cation, a quaternary ammonium cation, a quaternary phosphonium cation, a sulphonium cation, or any combination thereof. Preferably, the one or more cations comprise one or more cations selected from the following structures: 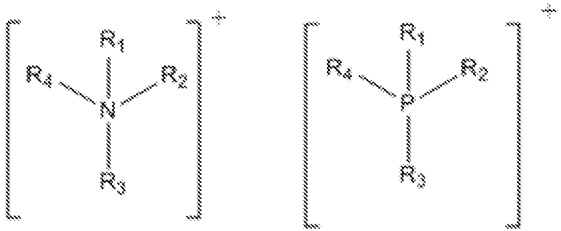 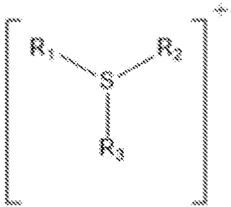 wherein R₁, R₂, R₃ and R₄ are independently selected from C₁ to C₃₀ straight chain or branched alkyl and alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl. More preferably, R₁, R₂, R₃ and R₄ are C₁ to C₃₀ straight chain or branched alkyl and alkenyl groups, such as C₁ to C₃₀ straight chain or branched alkyl groups. Most preferably, R₁, R₂, R₃ and R₄ are C₁ to C₁₈ straight chain or branched alkyl groups, such as C₁ to C₁₈ straight chain alkyl groups.

In some instances, the one or more cations comprise one or more cations selected from the following structures: 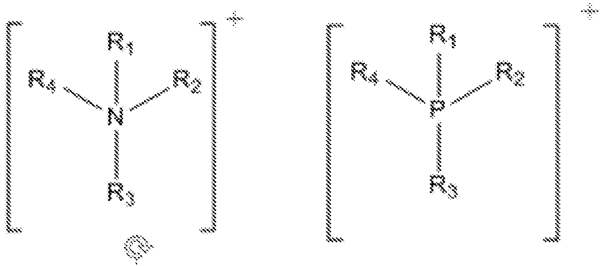 wherein R₁ to R₄ are as defined above, and wherein R₁, R₂ and R₃ are the same and R₄ is different to R₁, R₂ and R₃.

In other instances, the one or more cations comprise one or more cations selected from the following structure: 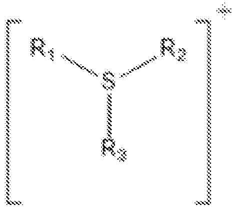 wherein R₁ to R₃ are as defined above, wherein R₁ and R₂ are the same and R₃ is different to R₁ and R₂.

In one highly preferred instance, the one or more cations comprise one or more cations selected from the following structures: 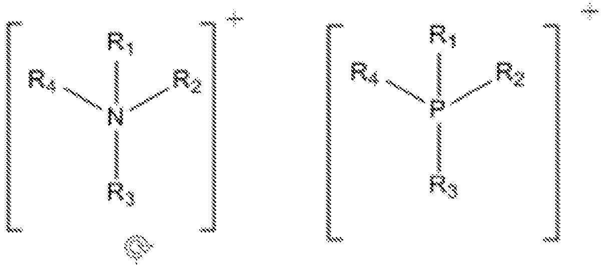 wherein R₁, R₂ and R₃ are C₄ to C₁₀ straight chain alkyl and wherein R₄ is C₁ to C₄ straight chain alkyl. Preferably, R₁, R₂ and R₃ are octyl and R₄ is methyl. Most preferably, the one or more cations comprise a phosphonium cation.

In another highly preferred instance, the one or more cations comprise one or more cations selected from the following structures: 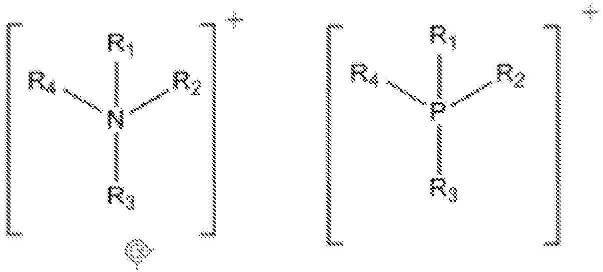 wherein R₁ and R₂ are C₁₄ to C₂₀ straight chain alkyl and wherein R₃ and R₄ are C₁ to C₄ straight chain alkyl. Preferably, R₁ and R₂ are octadecyl and R₃ and R₄ are methyl. More preferably, the one or more cations comprise an ammonium cation.

Accordingly, in highly preferable instances of the disclosure, the ionic liquid comprises a compound of the following formula: 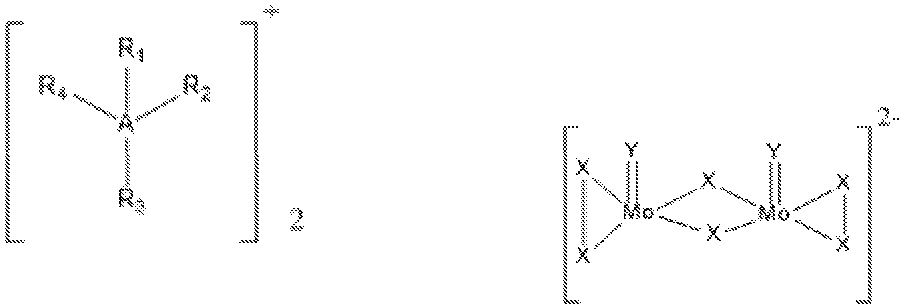 wherein (i) R₁, R₂ and R₃ are octyl; wherein R₄ is methyl and wherein A is P; or (ii) wherein R₁ and R₂ are octadecyl; wherein R₃ and R₄ are methyl and wherein A is N.

In other instances, the one or more cations comprise a substituted quaternary imidazolium cation comprising at least one ester group, or one or more cations selected from the following structures: 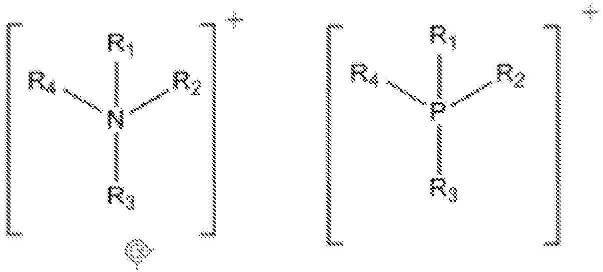 wherein at least one of R₁, R₂, R₃ and R₄ have the formula -R₅O(C=O)R₆ or -R₅(C=O)-O-R₆, wherein R₅ is a C₁ to C₁₀ straight chain orbranched alkyl or alkenyl group, or a C₃ to C₆ cycloalkyl or cycloalkenyl group; and R₆ is a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and-OC(O)(C₁ to C₆)alkyl;
and wherein the other of R₁, R₂, R₃ and R₄ that are not as defined above are independently selected from C₁ to C₃₀ straight chain or branched alkyl and alkenyl groups such as C₁ to C₁₀ alkyl or alkenyl groups; C₃ to C₆ cycloalkyl groups: C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl; or wherein the one or more cations comprise one or more cations selected from the following structure: 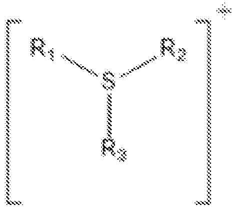 wherein at least one of R₁, R₂ and R₃ have the formula -R₅O(C=O)R₆ or -R₅(C=O)-O-R₆, wherein R₅ is a C₁ to C₁₀ straight chain or branched alkyl or alkenyl group, or a C₃ to C₆ cycloalkyl or cycloalkenyl group; and R₆ is a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and-OC(O)(C₁ to C₆)alkyl;
and wherein the other of R₁, R₂ and R₃ that are not as defined above are independently selected from C₁ to C₃₀ straight chain orbranched alkyl and alkenyl groups such as C₁ to C₁₀ alkyl or alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl. Preferably, the cation comprises a quaternary ammonium cation or a quaternary phosphonium cation.

In other instances, the one or more cations comprise a substituted quaternary imidazolium cation comprising at least one amide group, or one or more cations selected from the following structures: 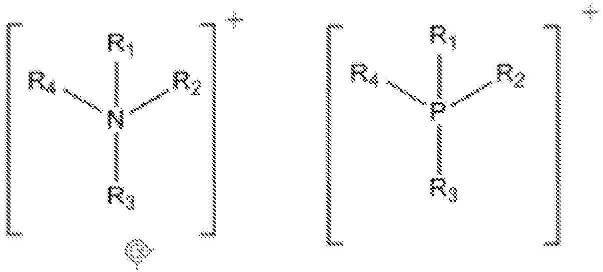 wherein at least one of R₁, R₂, R₃ and R₄ have the formula -R₅-N(R₇)-(C=O)R₆ or -R₅(C=O)-N(R₇)-R₆, wherein R₅ and R₆ are as defined in the paragraph above, and wherein R₇ is selected from H; a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl;
and wherein the other of R₁, R₂, R₃ and R₄ that are not as defined above are independently selected from C₁ to C₃₀ straight chain or branched alkyl and alkenyl groups such as C₁ to C₁₀ alkyl or alkenyl groups; C₃ to C₆ cycloalkyl groups: C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl; or wherein the one or more cations comprise one or more cations selected from the following structure: 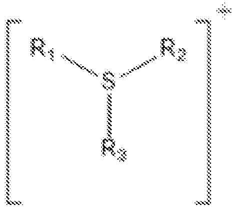 wherein at least one of R₁, R₂ and R₃ have the formula -R₅-N(R₇)-(C=O)R₆ or -R₅(C=O)-N(R₇)-R₆, wherein R₅ and R₆ are as defined above, and wherein R₇ is selected from H; a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl;
and wherein the other of R₁, R₂ and R₃ that are not as defined above are independently selected from C₁ to C₃₀ straight chain or branched alkyl and alkenyl groups such as C₁ to C₁₀ alkyl or alkenyl groups; C₃ to C₆ cycloalkyl groups: C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl. Preferably, the cation comprises a quaternary ammonium cation or a quaternary phosphonium cation.

In the instances described above, the one or more cations preferably comprise one or more cations selected from the following structures: 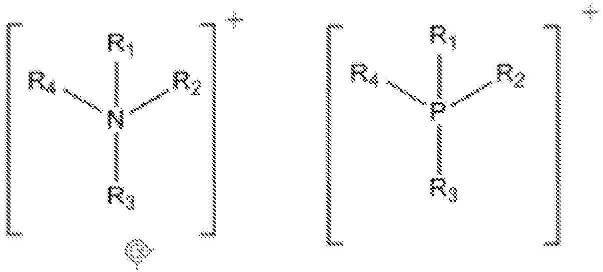 wherein three of R₁ R₂, R₃ and R₄ have the formula -R₅O(C=O)R₆; -R₅(C=O)-O-R₆; -R₅-N(R₇)-(C=O)R₆; or -R₅(C=O)-N(R₇)-R₆; wherein R₅, R₆ and R₇ are as defined above. Preferably, three of R₁ R₂, R₃ and R₄ have the formula -R₅O(C=O)R₆. Preferably, the cation comprises a quaternary ammonium cation; R₁ R₂, and R₃ have the formula -R₅O(C=O)R₆ wherein R₅ is C₂H₄; and R₄ is C₁ to C₅ alkyl, more preferably methyl.

Examples of cations discussed above include compounds known as ester quats, or amide quats. Examples of these are as follows:
Tri-alkanolamine esterquats of the formula: 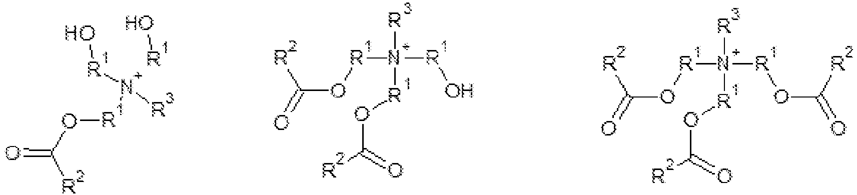 wherein R1 and R3 = a straight chain or branched C₁ to C₁₀ alkyl or alkenyl, or C₃ to C₆ cycloalkyl or cycloalkenyl, and wherein R2 = a C₁ to C₃₀ straight chain or branched alkyl or alkenyl group, or a or C₃ to C₆ cycloalkyl or cycloalkenyl group.
D-alkanolamine esterquats of the formula: 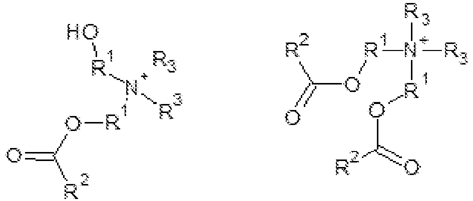 wherein R1 and R3 = a straight chain or branched C₁ to C₁₀ alkyl or alkenyl, or C₃ to C₆ cycloalkyl or cycloalkenyl, and wherein R2 = a C₁ to C₃₀ straight chain or branched alkyl or alkenyl group, or a or C₃ to C₆ cycloalkyl or cycloalkenyl group.
Diamidoamine quats of the formula: 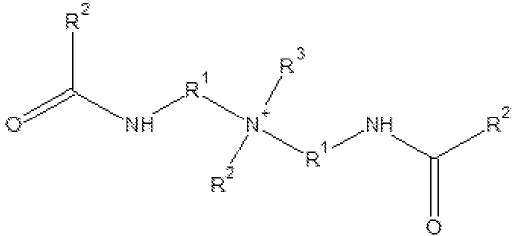 wherein R1 and R3 = a straight chain or branched C₁ to C₁₀ alkyl or alkenyl, or C₃ to C₆ cycloalkyl or cycloalkenyl, and wherein R2 = a C₁ to C₃₀ straight chain or branched alkyl or alkenyl group, or a or C₃ to C₆ cycloalkyl or cycloalkenyl group.

In other instances, the cations of the ionic liquids of the present disclosure may comprise quaternary imidazolium cations. For example, the cations of the ionic liquids of the disclosure may comprise imidazolium rings, wherein the imidazolium ring is substituted with one or more hydrocarbyl moieties such as alkyl, alkenyl and cycloalkyl and cycloalkenyl moieties, for example C₁ to C₃₀ alkyl or alkenyl moieties, or C₃ to C₇ cycloalkyl or cycloalkenyl moieties. In some instances, said hydrocarbyl moieties may themselves be substituted or interrupted with one or more moieties comprising heteroatoms.

In preferable embodiments, the imidazolium ring of the cation is substituted at the 1, 2 or 3 position, and preferably at the 1, 2 and 3 position. More preferably, the quaternary imidazolium cation is substituted at the 1, 2 and 3 positions of the imidazolium ring with one or more hydrocarbyl moieties such as alkyl, alkenyl and cycloalkyl and cycloalkenyl moieties, which may themselves be substituted or optionally interrupted with functional groups comprising heteroatoms.

In preferable embodiments, the substituted quaternary imidazolium cations comprise at least one amide group or at least one ester group.

An example of imidazolium cations of the present disclosure are the following quaternary imidazolium cations defined by the following formula: 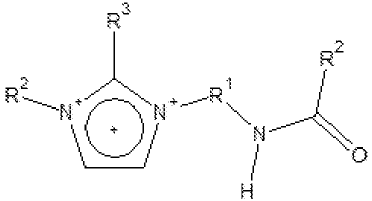 wherein R1 and R3 = a straight chain or branched C₁ to C₁₀ alkyl or alkenyl, or C₃ to C₆ cycloalkyl or cycloalkenyl, and wherein R2 = a C₁ to C₃₀ straight chain or branched alkyl or alkenyl group, or a or C₃ to C₆ cycloalkyl or cycloalkenyl group.

In some instances, the ionic liquids of the present disclosure may comprise one or more cationic species selected from: ammonium, azaannulenium, azathiazolium, benzimidazolium, benzofuranium, benzothiophenium, benzotriazolium, borolium, cinnolinium, diazabicyclodecenium, diazabicyclononenium, 1,4-diazabicyclo[2.2.2]octanium, diazabicyclo-undecenium, dibenzofuranium, dibenzothiophenium, dithiazolium, dithiazolium, furanium, guanidinium, imidazolium, indazolium, indolinium, indolium, morpholinium, oxaborolium, oxaphospholium, oxazinium, oxazolium, iso-oxazolium, , oxazolinium, pentazolium oxothiazolium, phospholium, phosphonium, phthalazinium, piperazinium, piperidinium, pyranium, pyrazinium, pyrazolium, pyridazinium, pyridinium, pyrimidinium, pyrrolidinium, pyrrolium, quinazolinium, quinolinium, isoquinolinium, quinoxalinium, quinuclidinium, selenazolium, sulfonium, tetrazolium, thiadiazolium, iso-thiadiazolium, thiazinium, thiazolium, iso-thiazolium, thiophenium, thiuronium, triazadecenium, triazinium, triazolium, iso-triazolium, and uranium, or a combination thereof.

For example, in an embodiment, the cation may be selected from the guanidinium, cyclic guanidinium, uronium, cyclic uronium, thiuronium and cyclic thiuronium cations. For example, cations of the following formula: 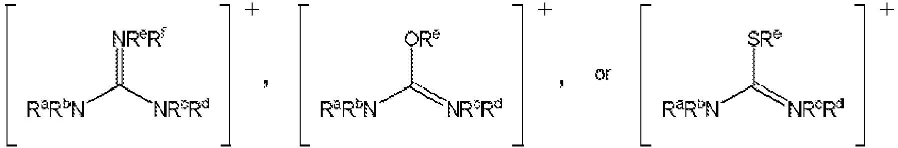 wherein: R^{a}, R^{b}, R^{c}, R^{d}, R^{e}, and R^{f} are each independently selected from a C₁ to C₃₀, straight chain orbranchedalkylgroup, a C₃ to C₈ cycloalkyl group, or a C₆ to C₁₀ aryl group, or any two of R^{a}, R^{b}, R^{c}, and R^{d}, attached to different nitrogen atoms form a methylene chain -(CH₂)_{q}- wherein q is from 2 to 5; wherein said alkyl, cycloalkyl or aryl groups or said methylene chain are unsubstituted or may be substituted by one to three groups selected from: C₁ to C₆ alkoxy, C₂ to C₁₂ alkoxyalkoxy, C₃ to C₈ cycloalkyl, C₆ to C₁₀ aryl, C₇ to C₁₀ alkaryl, C₇ to C₁₀ aralkyl, -CN, -OH, -SH, -NO₂, -CO₂R^{x}, -OC(O)R^{x}, -C(O)R^{x}, -C(S)Rₓ, -CS₂Rₓ, -SC(S)Rₓ, -S(O)(C₁ to C₆)alkyl, -S(O)O(C₁ to C₆)alkyl, -OS(O)(C₁ to C₆)alkyl, -S(C₁ to C₆)alkyl, -S-S(C₁ to C₆ alkyl), -NR^{x}C(O)NR^{y}R^{z}, -NR^{x}C(O)OR^{y}, -OC(O)NR^{y}R^{z},-NR^{x}C(S)OR^{y}, -OC(S)NR^{y}R^{z}, -NR^{x}C(S)SR^{y}, -SC(S)NR^{y}R^{z}, -NR^{x}C(S)NR^{y}R^{z}, -C(O)NR^{y}R^{z}, -C(S)NR^{y}R^{z}, -NR^{y}R^{z}, or a heterocyclic group, wherein R^{x}, R^{y} and R^{z} are independently selected from hydrogen or C₁ to C₆ alkyl.

Specific examples of guanidinium, uronium, and thiuronium cations suitable for use according to the present invention include:

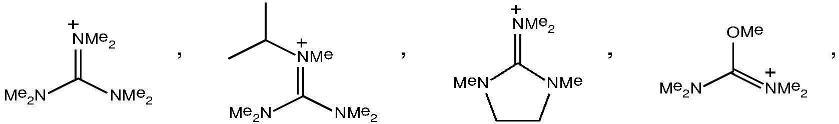

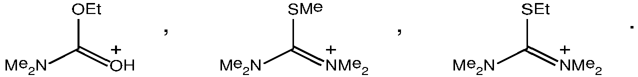

In a further embodiment, the cation comprises a cation comprising an electron-rich sulfur or selenium moiety. Examples include cations as defined above comprising pendant thiol, thioether, or disulfide substituents.

In another embodiment, the cation comprises an aromatic heterocyclic cationic species selected from: benzimidazolium, benzofuranium, benzothiophenium, benzotriazolium, cinnolinium, diazabicyclodecenium, diazabicyclononenium, diazabicyclo-undecenium, dithiazolium, imidazolium, indazolium, indolinium, indolium, oxazinium, oxazolium, *iso*-oxazolium, oxathiazolium, phthalazinium, pyrazinium, pyrazolium, pyridazinium, pyridinium, pyrimidinium, quinazolinium, quinolinium, *iso*-quinolinium, quinoxalinium, tetrazolium, thiadiazolium, *iso-*thiadiazolium, thiazinium, thiazolium, *iso*-thiazolium, triazinium, triazolium, and iso-triazolium.

For example, the cation may comprise: 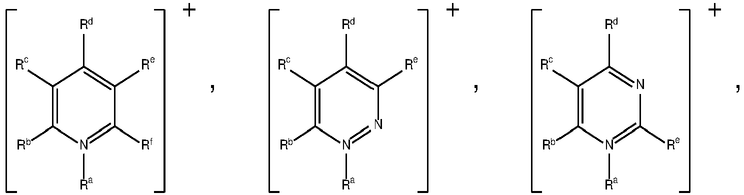 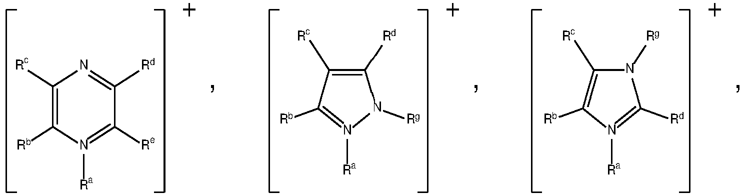 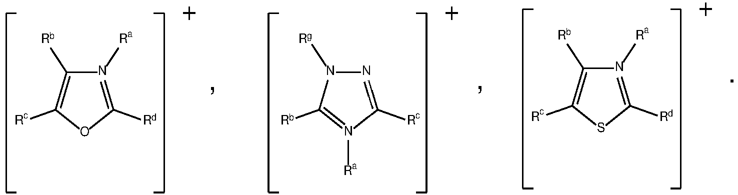 wherein: R^{a}, R^{b}, R^{c}, R^{d}, R^{e}, R^{f} and R^{g} are each independently selected from hydrogen, a C₁ to C₃₀, straight chain or branched alkyl group, a C₃ to C₈ cycloalkyl group, or a C₆ to C₁₀ aryl group, or any two of R^{b}, R^{c}, R^{d}, R^{e} and R^{f} attached to adjacent carbon atoms form a methylene chain - (CH₂)_{q}- wherein q is from 3 to 6; and wherein said alkyl, cycloalkyl or aryl groups or said methylene chain are unsubstituted or may be substituted by one to three groups selected from: C₁ to C₆ alkoxy, C₂ to C₁₂ alkoxyalkoxy, C₃ to C₈ cycloalkyl, C₆ to C₁₀ aryl, C₇ to C₁₀ alkaryl, C₇ to C₁₀ aralkyl, -CN, -OH, -SH, -NO₂, -CO₂R^{x}, -OC(O)R^{x}, -C(O)R^{x}, -C(S)R^{x}, -CS₂R^{x}, -SC(S)R^{x}, -S(O)(C₁ to C₆)alkyl, -S(O)O(C₁ to C₆)alkyl, -OS(O)(C₁ to C₆)alkyl, -S(C₁ to C₆)alkyl, -S-S(C₁ to C₆ alkyl), -NR^{x}C(O)NR^{y}R^{z}, -NR^{x}C(O)OR^{y}, -OC(O)NR^{y}R^{z}, -NR^{x}C(S)OR^{y}, -OC(S)NR^{y}R^{z}, -NR^{x}C(S)SR^{y}, -SC(S)N R^{y}R^{z}, -NR^{x}C(S)NR^{y}R^{z}, -C(O)NR^{y}R^{z}, -C(S)NR^{y}R^{z}, -NR^{y}R^{z}, or a heterocyclic group, wherein R^{x}, R^{y} and R^{z} are independently selected from hydrogen or C₁ to C₆ alkyl.

R^{a} is preferably selected from C₁ to C₃₀, linear or branched, alkyl, more preferably C₂ to C₂₀ linear or branched alkyl, still more preferably, C₂ to C₁₀ linear or branched alkyl, and most preferably C₄ to C₈ linear or branched alkyl. Further examples include wherein R^{a} is selected from methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl and n-octadecyl.

In the cations comprising an R^{g} group, R^{g} is preferably selected from C₁ to C₁₀ linear or branched alkyl, more preferably, C₁ to C₅ linear or branched alkyl, and most preferably R^{g} is a methyl group. In the cations comprising both an R^{a} and an R^{g} group, R^{a} and R^{g} are each preferably independently selected from C₁ to C₃₀, linear or branched, alkyl, and one of R^{a} and R^{g} may also be hydrogen. More preferably, one of R^{a} and R^{g} may be selected from C₂ to C₂₀ linear or branched alkyl, still more preferably, C₂ to C₁₀ linear or branched alkyl, and most preferably C₄ to C₈ linear or branched alkyl, and the other one of R^{a} and R^{g} may be selected from C₁ to C₁₀ linear or branched alkyl, more preferably, C₁ to C₅ linear or branched alkyl, and most preferably a methyl group. In a further preferred embodiment, R^{a} and R^{g} may each be independently selected, where present, from C₁ to C₃₀ linear or branched alkyl and C₁ to C₁₅ alkoxyalkyl. In some embodiments, one of R^{a} and R^{g} may be substituted with hydroxyl, methoxy or ethoxy.

In further embodiments, R^{b}, R^{c}, R^{d}, R^{e}, and R^{f} are independently selected from hydrogen and C₁ to C₅ linear or branched alkyl, and more preferably R^{b}, R^{c}, R^{d}, R^{e}, and R^{f} are hydrogen. In this embodiment, the cation preferably comprises a cation selected from: 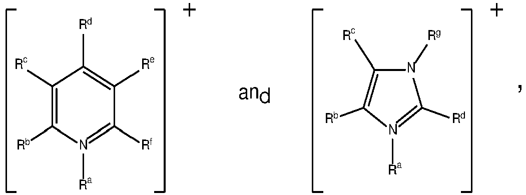 wherein: R^{a}, R^{b}, R^{c}, R^{d}, R^{e}, R^{f}, and R^{g} are as defined above. In preferable instances of this embodiment, the cation comprises a cation selected from: 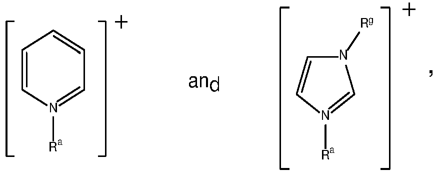 wherein: R^{a} and R^{g} are as defined above.

In further embodiments, the cation may comprise a cation selected from: 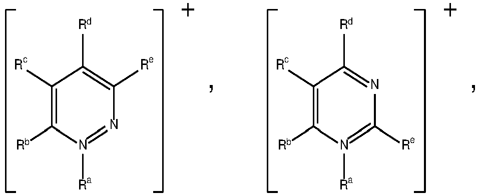 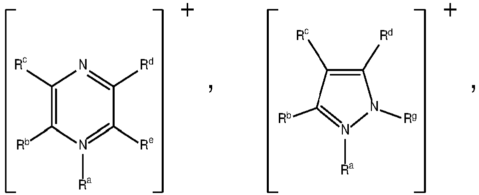 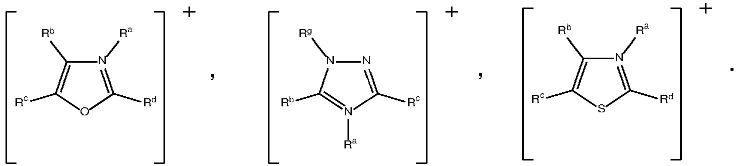 wherein: R^{a}, R^{b}, R^{c}, R^{d}, R^{e}, R^{f} and R^{g} are as defined above.

Specific examples of nitrogen-containing aromatic heterocyclic cations that may be used in accordance with this embodiment include:

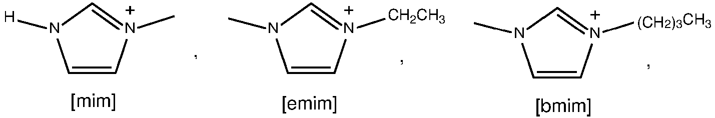

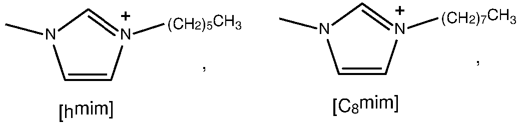

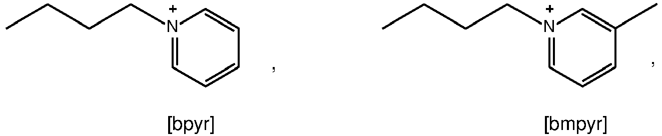

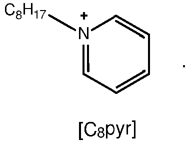

In further embodiments, the cation comprises a saturated heterocyclic cation having the formula: 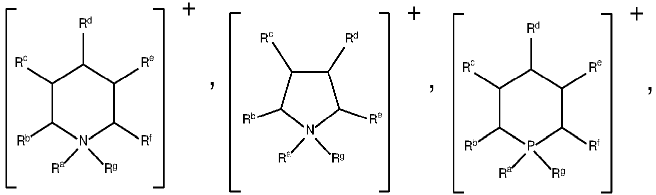 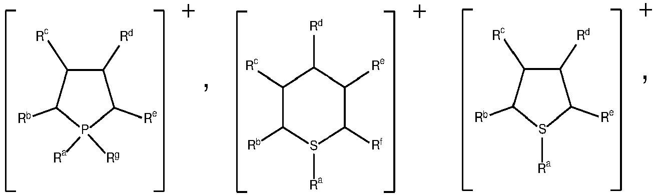 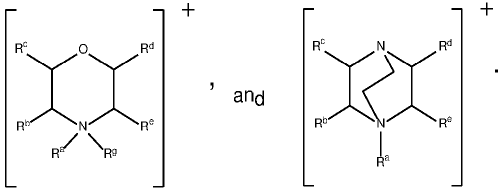 wherein: R^{a}, R^{b}, R^{c}, R^{d}, R^{e}, R^{f}, and R^{g} are as defined above. In preferable instances of this emobodiment, the cation comprises a saturated heterocyclic cation having the formula: 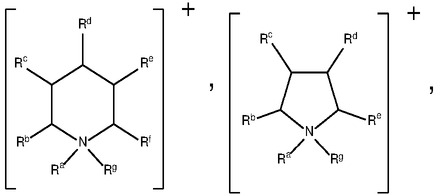 and is most preferably 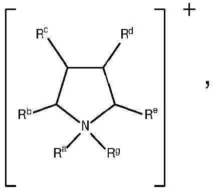 wherein: R^{a}, R^{b}, R^{c}, R^{d}, R^{e}, R^{f}, and R^{g} are as defined above.

A specific example of a preferred saturated heterocyclic cation suitable for use according to the present invention is 1-butyl-1-methylpyrrolidinium cation:

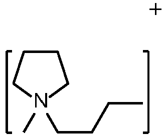

In further embodiments, the cation may comprise a saturated heterocyclic cation selected from: 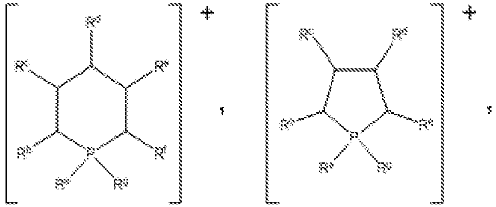 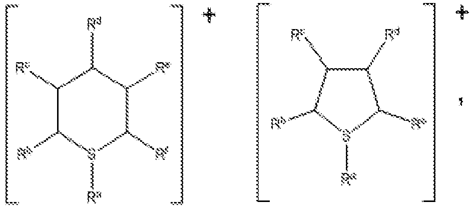 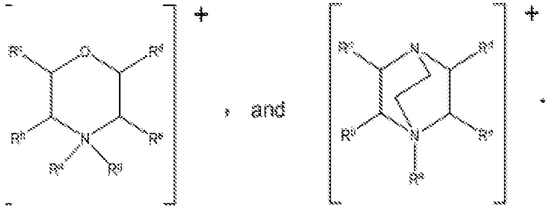 wherein: R^{a}, R^{b}, R^{c}, R^{d}, R^{e}, R^{f}, and R^{g} are as defined above.

In the saturated heterocyclic cations above, R^{a} is preferably selected from C₁ to C₃₀, linear or branched, alkyl, more preferably C₂ to C₂₀ linear or branched alkyl, still more preferably, C₂ to C₁₀ linear or branched alkyl, and most preferably C₄ to C₈ linear or branched alkyl. Further examples include wherein R^{a} is selected from methyl, ethyl, n-propyl, n-butyl, n-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl and n-octadecyl.

In the saturated heterocyclic cations comprising an R^{g} group, R^{g} is preferably selected from C₁ to C₁₀ linear or branched alkyl, more preferably, C₁ to C₅ linear or branched alkyl, and most preferably R^{g} is a methyl group.

In the saturated heterocyclic cations comprising both an R^{a} and an R^{g} group, R^{a} and R^{g} are each preferably independently selected from C₁ to C₃₀, linear or branched, alkyl, and one of R^{a} and R^{g} may also be hydrogen. More preferably, one of R^{a} and R^{g} may be selected from C₂ to C₂₀ linear or branched alkyl, still more preferably, C₂ to C₁₀ linear or branched alkyl, and most preferably C₄ to C₈ linear or branched alkyl, and the other one of R^{a} and R^{g} may be selected from C₁ to C₁₀ linear or branched alkyl, morepreferably, C₁ to C₅ linear or branched alkyl, and most preferably a methyl group. In a further preferred embodiment, R^{a} and R^{g} may each be independently selected, where present, from C₁ to C₃₀ linear or branched alkyl and C₁ to C₁₅ alkoxyalkyl.

In further embodiments, R^{b}, R^{c}, R^{d}, R^{e}, and R^{f} are independently selected from hydrogen and C₁ to C₅ linear or branched alkyl, and more preferably R^{b}, R^{c}, R^{d}, R^{e}, and R^{f} are hydrogen.

The ionic liquids of the present disclosure are preferably liquid at a temperature of 100°C and above, preferably 50°C and above. More preferably, the ionic liquids of the present disclosure are liquid at a temperature of 25°C and above, preferably 20°C and above, more preferably 10°C and above, and most preferably 0°C and above.

An advantage of the ionic liquids of the present disclosure is that they can be synthesised such that they comprise less than 5 wt% halide, preferably less than 2.5 wt. % halide, and most preferably less than 1 wt. % halide. More preferably, the ionic liquids comprise less than 0.5 wt. % halide, and preferably less than 0.1 wt. % halide. Most preferably, the ionic liquids of the present disclosure are free of halide.

The lubricant compositions such as lubricating oil compositions or grease compositions of the present disclosure comprise one or more ionic liquids as described above and one or more base oils. lubricant compositions such as lubricating oil compositions and grease compositions of the present disclosure may comprise any suitable amount of one or more base oils and any suitable amount of one or more ionic liquids as described herein.

Typically, the lubricant compositions such as lubricating oil composition or grease composition comprises the one or more ionic liquids in an amount of from 0.05 wt. % to 20 wt. %, preferably from 0.1 wt. % to 10 wt. %, and most preferably from 0.1 wt. % to 5 wt. %. Preferably, the composition comprises the one or more ionic liquids in an amount of from 0.5 wt. % to 1.5 wt. %.

The base oils employed in the lubricant compositions of the present disclosure are typically oils used in automotive and industrial applications such as, among others, turbine oils, hydraulic oils, gear oils, crankcase oils and diesel oils. The base stock may comprise at least 90%, or at least 95% by weight of the total lubricant composition.

Typical lubricant basestocks that can be used in the lubricant compositions such as lubricating oil or grease compositions of the disclosure may include natural base oils, including mineral oils, petroleum oils, paraffinic oils and vegetable oils, as well as oils derived from synthetic sources.

In particular, lubricant basestocks that can be used in this invention may be petroleum-based or synthetic stocks including any fluid that falls into the API basestock classification as Group I, Group II, Group III, Group IV, and Group V. The hydrocarbon base oil may be selected from naphthenic, aromatic, and paraffinic mineral oils.

Suitable synthetic oils may also be selected from, among others, ester-type oils (such as silicate esters, pentaerythritol esters and carboxylic acid esters), esters, diesters, polyol esters, polyalphaolefins (also known as PAOS or poly-a.-olefins), hydrogenated mineral oils, silicones, silanes, polysiloxanes, alkylene polymers, polyglycol ethers, polyols, bio-based lubricants and/or mixtures thereof.

Preferably, the one or more base oils comprise one or more base stocks selected from Group I base stocks, Group II base stocks, Group III base stocks, Group IV base stocks and Group V base stocks; or the one or more base oils comprise one or more synthetic oils selected from polyalpha-olefins, synthetic esters, polyalkylene glycols, phosphate esters, alkylated naphthalenes, silicate esters, ionic fluids, or multiply alkylated cyclopentanes.

Typically, the one or more base oils are present in the lubricant compositions such as lubricating oil composition or grease composition in an amount of from 50% to 99% by weight of the lubricant composition. Preferably, the one or more base oils are present in the lubricant composition in an amount of from 75% to 99%. The amount of one or more base oils that are included in the composition will vary depending on the type of lubricant composition such as whether the composition is a lubricating oil composition or a grease composition, as discussed in further detail below. Typically, where the composition is a lubricating oil composition that composition will comprise a higher amount by weight of the one or more base oils than when the composition is a grease composition, since grease compositions comprise one or more thickeners, as discussed in further detail below.

Lubricant compositions of the present disclosure typically further comprise one or more additives optionally selected from one or more pour point additives, anti-foaming additives, viscosity index improvers, antioxidants, detergents, corrosion inhibitors, anti-wear additives, friction modifiers, extreme pressure additives, or any combination thereof.

The additives may be present in compositions of the disclosure in any suitable amount. Typically, the additives are present in an amountof from 0.1 wt. % to 35 wt. % of the composition, preferably from 1 wt. % to 20 wt. % and most preferably from 1 wt. % to 10 wt. %.

In some instances, the lubricant composition of the disclosure is a lubricating oil composition. The term lubricating oil composition as used herein is intended to refer to compositions suitable for lubricating the working parts of an internal combustion engine such as a spark ignition engine or compression ignition engine, or industrial machinery. Such lubricating oil compositions are compositions that are liquid at room temperature. The term lubricating oil composition is intended to cover all such oils. Lubricating oil compositions of the disclosure are typically liquid at 25°C in the absence of shear forces.

In other instances, the lubricant composition of the disclosure is a grease composition. The term grease composition as used herein is used to refer to compositions suitable for lubricating the working parts of an internal combustion engine such as a spark ignition engine or compression ignition engine, or industrial machinery, where the composition is solid at room temperature in the absence of shear forces. Grease compositions of the disclosure are thus typically solid at 25°C in the absence of shear forces. Such compositions typically comprise one or more base oils as discussed above and one or more thickeners. On the application of shear forces to the grease composition, the composition typically greatly reduces in viscosity to have a viscosity similar to or the same as the viscosity of the one or more base oils present in the grease composition.

Grease compositions of the disclosure thus comprise one or more ionic liquids as described above, one or base oils and one or more thickeners. Any suitable thickener known for use in grease compositions can be used. Examples of appropriate thickeners that can be used include soap such as an alkali metal or alkaline earth metal salt of a C₁₄ to C₂₀ fatty acid, or derivatives thereof; an alkaline earth metal sulphonate such as calcium sulphonate; a urea; a substituted urea; a polyurea; a substituted polyurea; clay; tar; graphite; micap; silica; or a combination thereof. Preferably, the one or more thickeners comprise soap such as an alkali metal or alkaline earth metal salt of a C₁₄ to C₂₀ fatty acid, or derivatives thereof.

The thickeners may be present in the grease compositions in any suitable amount. Typically, the one or more thickeners are present in the grease compositions in an amount of from 5% to 50% by weight of the composition. Preferably, the one or more thickeners are present in an amount of from 10% to 30% by weight of the composition.

Preferably, wherein the grease composition comprises from 50% to 95% by weight of one or more base oils; and from 5% to 50% by weight of one or more thickeners; preferably wherein the grease composition comprises from 10% to 30% by weight of one or more thickeners.

Any suitable lubricant base oil may be used in the grease compositions, such as those discussed above.

According to a second aspect of the invention, there is provided a method of producing a lubricant composition according to the first aspect of the invention, comprising the steps of combining one or more base oils with one or more ionic liquids, and optionally one or more additives or one or more thickeners. Any suitable methods known in the art may be used.

According to a third aspect of the invention, there is provided a method of operating industrial machinery, a spark ignition engine or a compression ignition engine comprising lubricating said engine with a lubricant composition according to the first aspect of the invention. Any suitable known methods of operating said industrial machinery and engines using said lubricants including the application of said lubricants to the engines may be used. It will be appreciated that specific compositions of the invention may be suitable for a certain use, whilst other compositions of the invention will be suitable for other uses.

According to a fourth aspect of the invention, there is provided an ionic liquid as described above in accordance with the first aspect of the invention.

According to a fifth aspect of the invention, there is provided the use of an ionic liquid according to the fourth aspect of the invention, in a lubricant composition, for the purpose of improving the anti-friction properties of the lubricant composition and/or for the purpose of improving the anti-wear properties of the lubricant composition.

Preferably, the lubricant composition is as described above in accordance with the first aspect of the invention. The ionic liquid may be used for the purpose of improving the anti-friction properties of the lubricant composition; or, the ionic liquid may be used for the purpose of improving the anti-wear properties of the lubricant composition. Accordingly, the ionic liquids may improve the anti-wear properties of the composition without improving the friction reducing properties of the composition. Alternatively, the ionic liquids may improve the friction reducing properties of the composition without improving the anti-wear properties of the composition. Preferably, the ionic liquids improve the anti-wear properties of the composition and the friction reducing properties of the composition.

The ionic liquids of the present disclosure are improved friction reducing and anti-wear additives when used in lubricant compositions when compared to many commercially available additives for such purposes. Without being limited by theory, this is believed to be, in part, because the ionic liquids exist as separate charged ions in the liquid state. When a lubricant composition is applied to moving parts, the composition forms a film between the surfaces of the moving parts. Moving parts are typically metal. Many currently commercially available anti-wear and friction reducing additives are neutral molecules and so are well dispersed in the base oil of the lubricant composition within the film between the moving parts. In contrast, because ionic liquids exist as separate charged ions in the liquid state, they adsorb onto the polar metal surface of the moving parts. As such there is a localised concentration of the ionic liquids at the metal oil interface of each surface. This localised concentration of ionic liquids forms a film on the surface of each moving part. Essentially, the ionic liquid additive forms a film within the film of lubricating base oil. The ionic liquid film upon the surface of the moving parts is thought to act as an anti-wear film, reducing wear upon the metal surfaces. The ionic liquid film in contact with the metal surfaces may also serve to reduce friction between the moving parts.

Surprisingly, compared to the ionic liquid additives disclosed in WO2021/005372, the ionic liquids of the present disclosure have been found to have improved solubility and stability when included in lubricant compositions.

The term "anti-friction properties" as used herein or "friction reducing properties of a lubricating oil composition" are used herein are well known terms of art that the skilled person will be familiar with. Such terms refer to the coefficient of friction when a lubricant composition is used in a lubricating application. The lower the coefficient of friction, the better a composition is as a lubricant and the better the anti-friction properties of the composition. Standard tests for measuring the coefficient of friction of a lubricating oil composition are known in the art. In an instance, the test used to measure coefficient of friction is ASTM D5183.

The term "anti-wear" properties as used herein or "wear reducing properties" with respect to a lubricant composition are well known terms of art that the skilled person will be familiar with. The wear properties of a lubricant composition are a measure of the extent to which surfaces to which the lubricant composition is applied are worn down in use. Wear can be measured, for example, by wear scar which is a distance measurement. The greater the wear scar distance, the more a surface has been worn down in use of lubricant composition. A lubricant composition with a lower wear scar distance measurement would be said to have improved anti-wear properties. Tests for measuring wear are known to the skilled person. In an instance, the wear properties of a lubricant composition can be measured by ASTM D4172 - 94 (2016).

According to a sixth aspect of the invention, there is provided an additive concentrate for use in a lubricant composition, wherein the additive concentrate comprises an ionic liquid according to the fourth aspect of the invention, and one or more further additives selected from one or more pour point additives, anti-foaming additives, viscosity index improvers, antioxidants, detergents, corrosion inhibitors, anti-wear additives, friction modifiers, extreme pressure additives, or any combination thereof.

According to a seventh aspect of the invention, there is provided a process for making an ionic liquid according to the fourth aspect of the invention, wherein the process comprises:
(a) providing an ionic liquid comprising one or more cations as described above and one or more anions of the following formula: 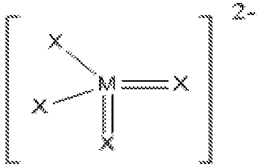 wherein each X is independently selected from O or S; and wherein M = Mo; and
(b) heating the ionic liquid from step (a) to a temperature of from 70°C to 130°C in the presence of air or oxygen so as to provide the ionic liquid according to the fourth aspect of the invention.

Preferably, the ionic liquid is heated to a temperature of from 80°C to 120°C.

Preferably, step (b) further comprises stirring the ionic liquid from step (a).

Preferably, the one or more anions of the ionic liquid in step (a) are of the following formula: 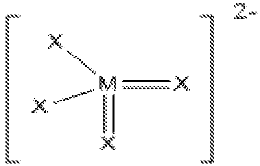 wherein each X is O; or wherein each X is S; preferably wherein each X is S.

Typically, step (b) of heating the ionic liquid is carried out for a timer period of from 5 hours to 20 hours, preferably from 8 hours to 16 hours, and most preferably for about 12 hours.

Typically, after step (b) has been carried out, the ionic liquid formed by heating step (b) is dried under vacuum. Typically, the vacuum drying is carried out at a temperature of from 80°C to 120°C, such as about 100°C.

The ionic liquid in step (a) may be synthesised using any suitable technique.

Typically, the ionic liquid in step (a) is prepared by stirring a suitable mixture of a salt comprising one or more cations as described above and a thiomolybdate salt comprising an MoS₄²⁻ anion in a suitable solvent. Typically, the salt comprising the cation and the thiomolybdate salt are mixed in a 2:1 molar ratio. Typically, the mixture is then stirred at a temperature of from 20°C to 60°C (for example around 50°C). The solvent is typically mixed for a time period of from 12 hours to 30 hours; preferably 20 hours to 28 hours such as around 24 hours.

After preparation, the ionic liquid comprising the one or more cations and the MoS₄²⁻ present in the solvent is typically treated so as to remove unwanted by-products and unreacted material, by a suitable method such as filtration. The solvent is then typically removed by a suitable technique such as heating the mixture to a temperature of from 40°C to 70°C for a time period of from 10 hours to 15 hours.

According to an eighth aspect of the invention, there is provided a process for making an ionic liquid according to the fourth aspect of the invention, wherein the process comprises
(a) forming a solution of a salt comprising one or more cations and one or more anions of the following formula: 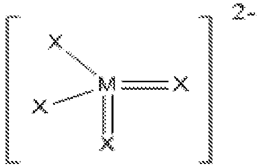 wherein each X is independently selected from O or S; and wherein M = Mo;
(b) heating the solution from step (a) to a temperature of from 70°C to 130°C in the presence of air or oxygen; preferably wherein the solution is heated to a temperature of from 80°C to 120°C;
(c) optionally, adding one or more bases to the solution; and
(d) contacting the solution with a salt comprising one or more cations as described herein and one or more anions to form the ionic liquid according to the fourth aspect of the invention.

Preferably, wherein step (a) comprises stirring the salt in a suitable solvent to form the solution.

Preferably, step (d) further comprises stirring the solution upon contacting.

Preferably, the salt added to the solution in step (d) is in the form of a solution.

Further details of the processes and synthetic techniques discussed above may be found in Z. anorg. Allg. Chem. 472, 69-74 (1981).

The ionic liquids of the present disclosure can also be synthesised by suitable methods known in the art for synthesising said ionic liquids. Examples of such methods include those discussed in Chem. Eur. J. 2009, 15, 12273 - 12282, although the skilled person will appreciate that other methods known in the art may be used.

### BRIEF DESCRIPTION OF THE DRAWINGS

Figure 1 depicts the infrared spectrum of ([P_{1,8,8,8}]₂MoS₄).
Figure 2 depicts the infrared spectrum of ([P_{1,8,8,8}]₂Mo₂O₂S₆).
Figure 3 depicts the UV-Vis spectrum of ([P_{1,8,8,8}]₂MoS₄).
Figure 4 depicts the UV-Vis spectrum of ([P_{1,8,8,8}]₂Mo₂O₂S₆).
Figure 5 depicts the infrared spectrum of ([P_{1,8,8,8}]₂Mo₂O₂S₆).
Figure 6 depicts the UV-Vis spectrum of ([P_{1,8,8,8}]₂Mo₂O₂S₆).
Figure 7 depicts the 1H NMR spectrum of ([P_{1,8,8,8}]₂MoS₄).
Figure 8 depicts the 1H NMR spectrum of ([P_{1,8,8,8}]₂Mo₂O₂S₆).
Figure 9 depicts the 1H NMR spectrum of ([P_{1,8,8,8}]₂Mo₂O₂S₆) after further purification by solvent based separation.
Figure 10 depicts the 1H NMR spectrum of ([P_{1,8,8,8}]₂Mo₂O₂S₆).
Figure 11 depicts the 13C NMR spectrum of ([P_{1,8,8,8}]₂MoS₄).
Figure 12 depicts the 13C NMR spectrum of ([P_{1,8,8,8}]₂Mo₂O₂S₆).
Figure 13 depicts the 13C NMR spectrum of ([P_{1,8,8,8}]₂Mo₂O₂S₆) after further purification by solvent based separation.
Figure 14 depicts the 13C NMR spectrum of ([P_{1,8,8,8}]₂Mo₂O₂S₆).
Figure 15 depicts the 95Mo NMR spectrum of ([P_{1,8,8,8}]₂MoS₄).
Figure 16 depicts the 95Mo NMR spectrum of ([P_{1,8,8,8}]₂Mo₂O₂S₆).
Figure 17 shows the coefficient of friction of the commercially available anti-friction additive MoDTC in a certain lubricating oil compared to the coefficient of friction of 6 batches of the ionic liquid [ P_{1,8,8,8}]₂ [Mo₂O₂S₆] in the same lubricating oil.
Figure 18 shows the compatibility data for the ionic liquid [P_{1,8,8,8}]₂ [Mo₂O₂S₆] produced by thermal treatment in various different lubricant oils.
Figure 19 shows the results of high temperature corrosion testing (according to ASTM D6594) for ([P P_{1,8,8,8}]₂[Mo₂O₂S₆]) (produced by thermal treatment) and the additives molybdenum dialkyl dithiocarbamate and molybdenum dialkyl dithiophosphate.
Figure 20 shows the results of high temperature corrosion testing (according to ASTM D6594) for ([P P_{1,8,8,8}]₂ [Mo₂O₂S₆]) where the ionic liquid is synthesised directly.
Figure 21 shows the results of high temperature corrosion testing (according to ASTM D6594) for the ionic liquid [N_{1,1,18,18}][Mo₂O₂S₆].

### DETAILED DESCRIPTION OF THE INVENTION

Examples 2 and 3 involve synthesis of ionic liquids of the invention. Examples 1 and 4 are reference examples.

### Example 1 - Synthesis of trioctylmethylphosphonium thiomolybdate [P_{1,8,8,8}]₂[MoS₄]

A metathesis reaction was carried out between trioctylmethylphosphonium chloride and ammonium tetrathiomolybdate in an organic solvent, followed by removal of the byproduct and solvent. The product was then vacuum dried. The product was characterised as discussed in further detail below.

### Example 2 - Synthesis of [P_{1,8,8,8}]₂[Mo₂O₂S₆] by thermal treatment of [P_{1,8,8,8}]₂[MoS₄]

A metathesis reaction was carried out between trioctylmethylphosphonium methylcarbonate and ammonium tetrathiomolybdate in an organic solvent, followed by removal of the byproduct and solvent. The product was then vacuum dried. The product was a viscous blackish-brown liquid crude product.

This product was further treated with ambient air at a temperature of 100°C with vigorous stirring under vacuum (800 mbar). The product was characterised as discussed in further detail below.

The ionic liquid product was further purified by solvent based separation using a silica column. The product was characterised as discussed in further detail below.

### Example 3 - Direct synthesis of [P_{1,8,8,8}]₂[Mo₂O₂S₆] without thermal treatment

Ammonium thiomolybdate was dissolved in deionised water and heated to 95°C while stirring for an hour. UV-Visible spectroscopy was then used to detect the conversion of the [MoS₄]²⁻ anion to [Mo₂O₂S₆]²⁻. The solution contained some precipitate. Potassium hydroxide was then added to the solution to dissolve the precipitate. The solution was then filtered and the filtrate added to a 30% trioctylmethlphosphonium methylcarbonate solution in methanol whilst stirring. The mixture was left to settle for two hours and separated into an ionic liquid layer and a water/methanol layer. The water/methanol layer was decanted off. The viscous ionic liquid layer was washed with water and isopropyl alcohol. The product was characterised as discussed in further detail below.

The ionic liquids produced in examples 2 and 3 showed improved solubility and stability than the ionic liquid of Example 1 when included in lubricant compositions.

### Example 4 - Synthesis of [P_{1,8,8,8}]₂[Mo₂O₂S₈]

Ammonium heptamolybdate tetrahydrate (2.5 g, 2.0 mmol) is dissolved in 100 mL of water. Then, 4 of sulfur is dissolved in 16 ml of a 20 % aqueous solution of ammonium sulfide and slowly added to the reaction. The reaction is stirred for 24 hours. The mixture is then filtered into a trioctylmethylphosphonium methylcarbonate solution in methanol (30%, 22 g) with vigorous stirring, and a deep red, viscous liquid separates immediately. The mixture was left to settle for 2 h, and the H₂O/MeOH layer was decanted off. The precipitate was extracted with acetonitrile (3 x 20 mL), filtered and the solvent was removed under vacuum. The product was isolated as deep-red viscous IL (4.2 g, FW: 1401.82 g/mol, 3 mmol, 60%).

### Characterisation of ionic liquids

The ionic liquids produced in Examples 1 to 3 were characterised by infrared, UV-Vis and NMR spectroscopy.

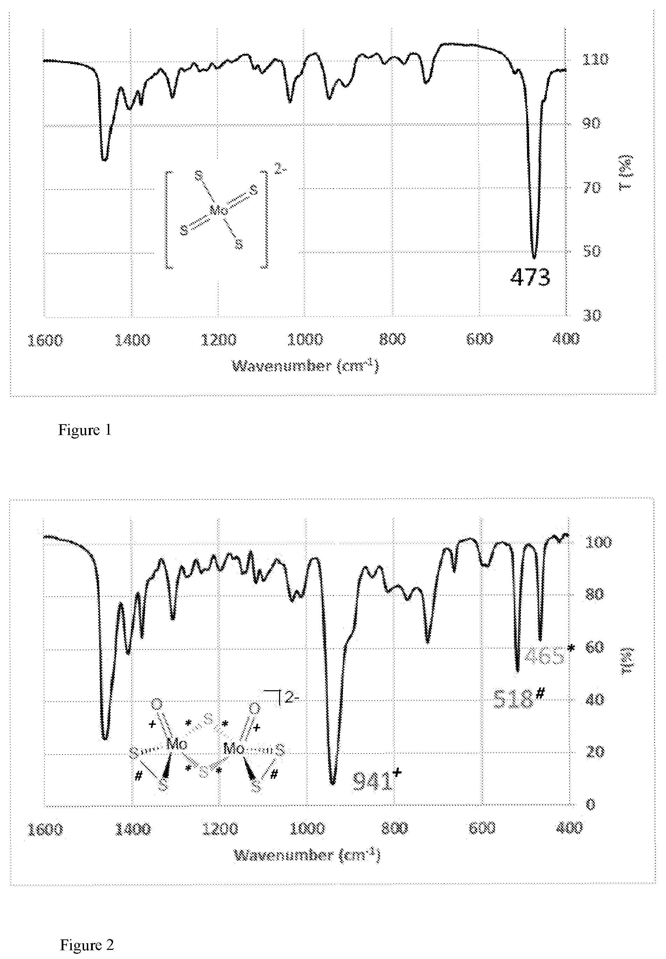

Figure 1 depicts the infrared spectrum of the product of Example 1 ([P_{1,8,8,8}]₂ [MoS4]).

Figure 2 depicts the infrared spectrum of the product of Example 2 ([P_{1,8,8,8}]₂ [Mo₂O₂S_{6]}).

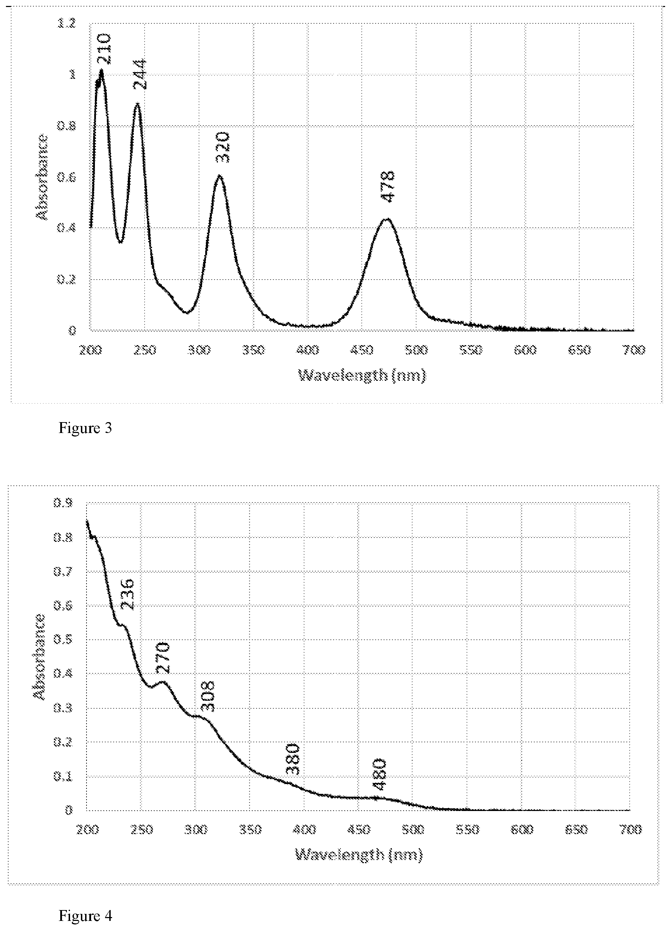

Figure 3 depicts the UV-Vis spectrum of the product of Example 1 ([P_{1,8,8,8}]₂ [MoS₄]).

Figure 4 depicts the UV-Vis spectrum of the product of Example 2 ([P_{1,8,8,8}]₂ [Mo₂O₂S₆]).

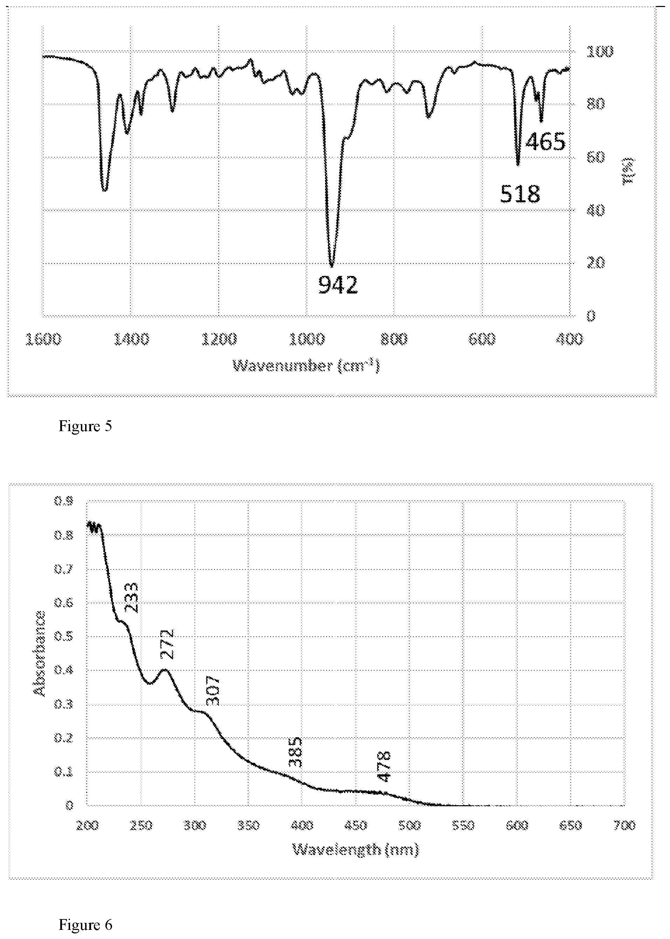

Figure 5 depicts the infrared spectrum of the product of Example 3 ([P_{1,8,8,8}]₂ [Mo₂O₂S₆]).

Figure 6 depicts the UV-Vis spectrum of the product of Example 3 ([P_{1,8,8,8}]₂ [Mo₂O₂S₆]).

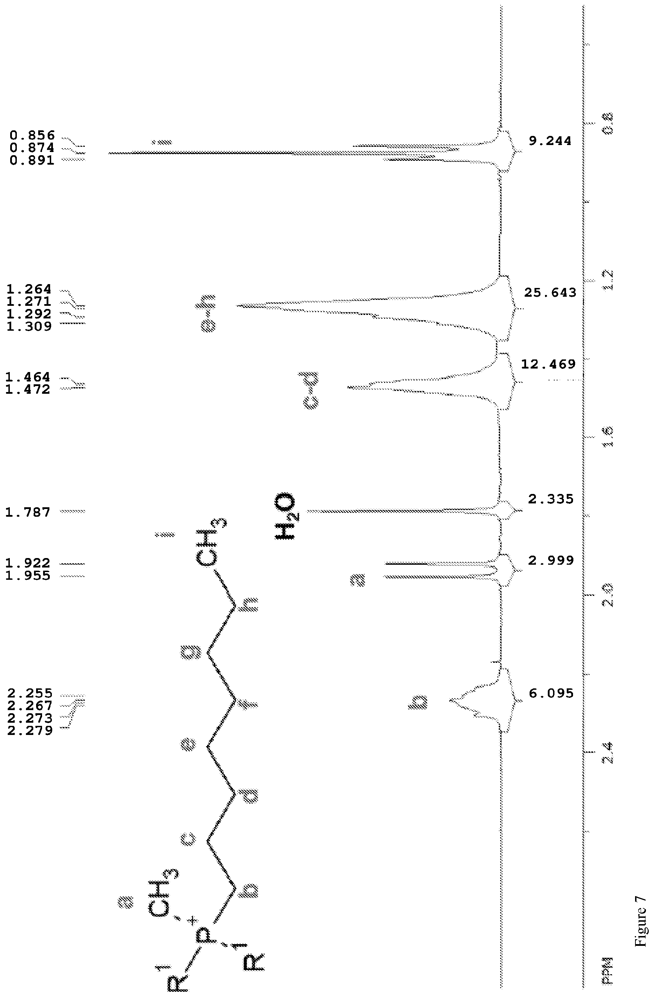

Figure 7 depicts the 1H NMR spectrum of the product of Example 1 ([P_{1,8,8,8}]₂ [MoS₄]).

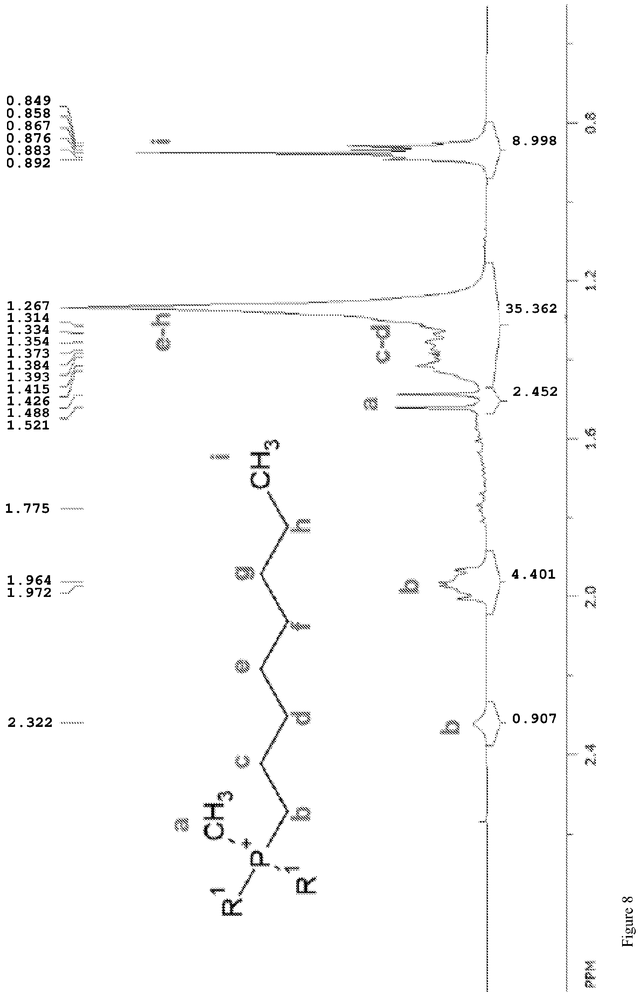

Figure 8 depicts the 1H NMR spectrum of the product of Example 2 ([P_{1,8,8,8}]₂ [Mo₂O₂S₆]).

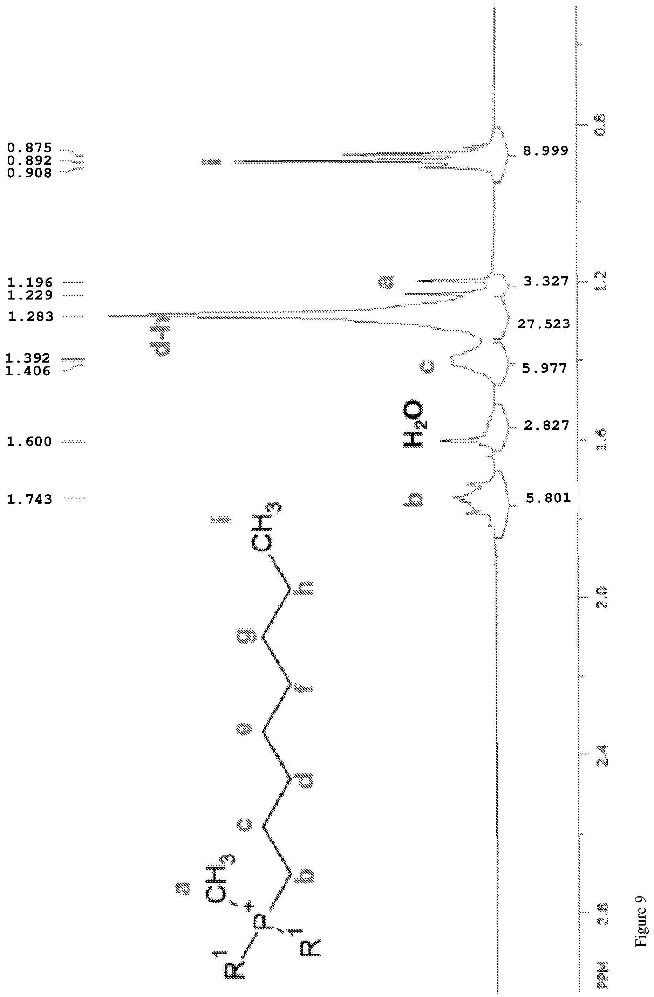

Figure 9 depicts the 1H NMR spectrum of the product of Example 2 ([P_{1,8,8,8}]₂ [Mo₂O₂S₆]) after further purification by solvent based separation.

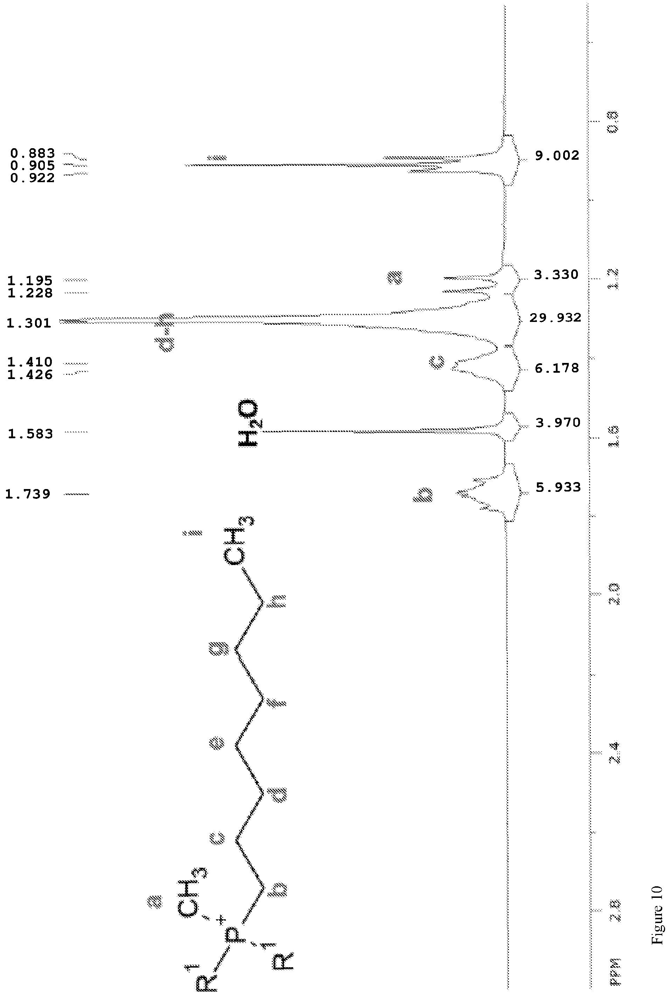

Figure 10 depicts the 1H NMR spectrum of the product of Example 3 ([P_{1,8,8,8}]₂ [Mo₂O₂S₆]).

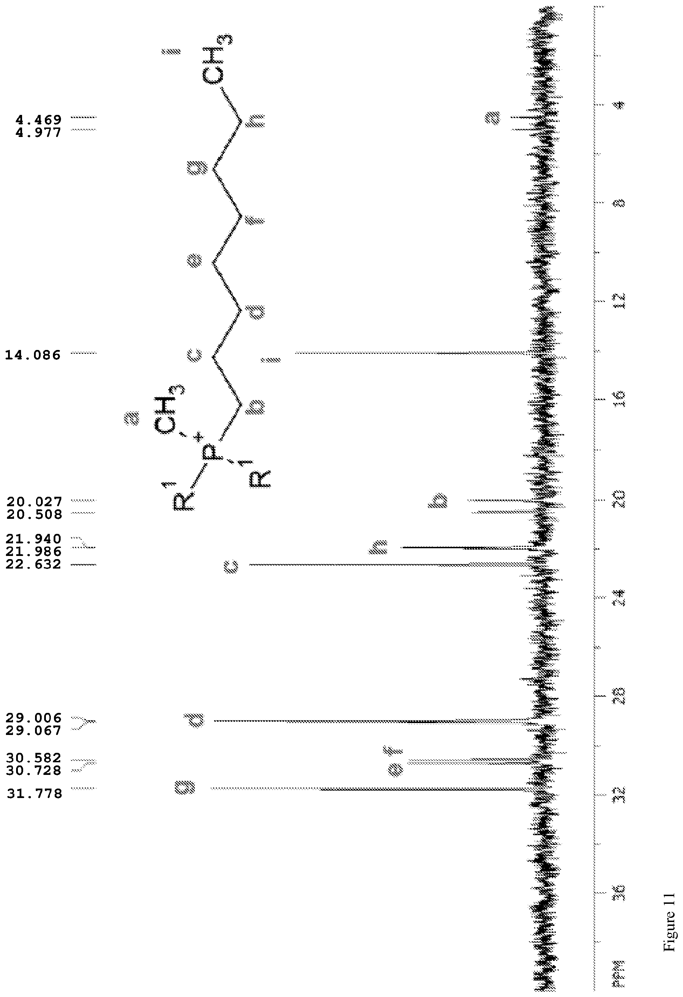

Figure 11 depicts the 13C NMR spectrum of the product of Example 1 ([P_{1,8,8,8}]₂ [MoS₄]).

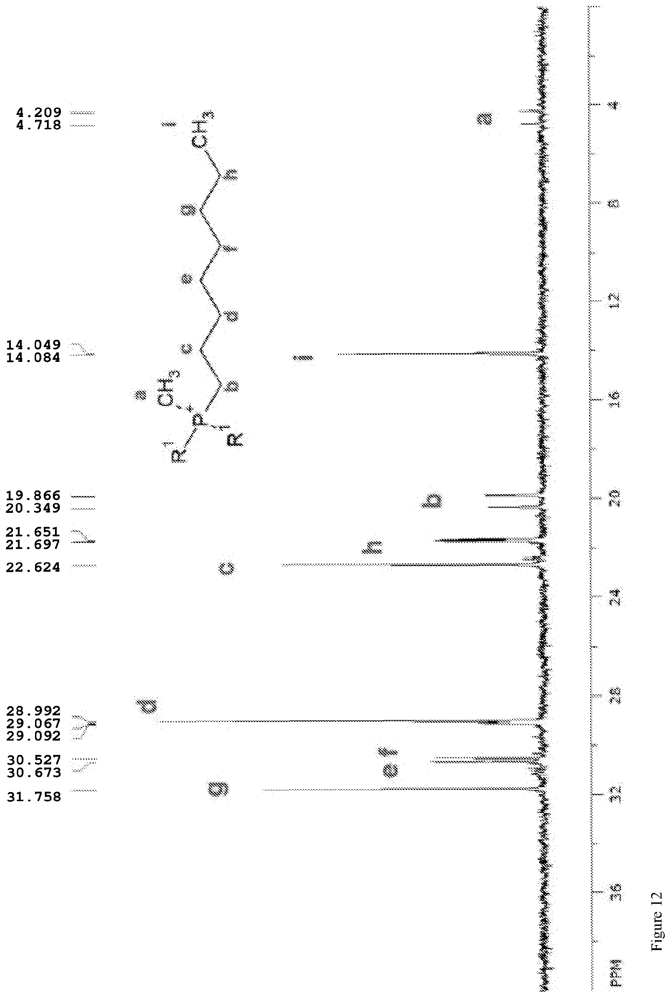

Figure 12 depicts the 13C NMR spectrum of the product of Example 2 ([P_{1,8,8,8}]₂ [Mo₂O₂S₆]).

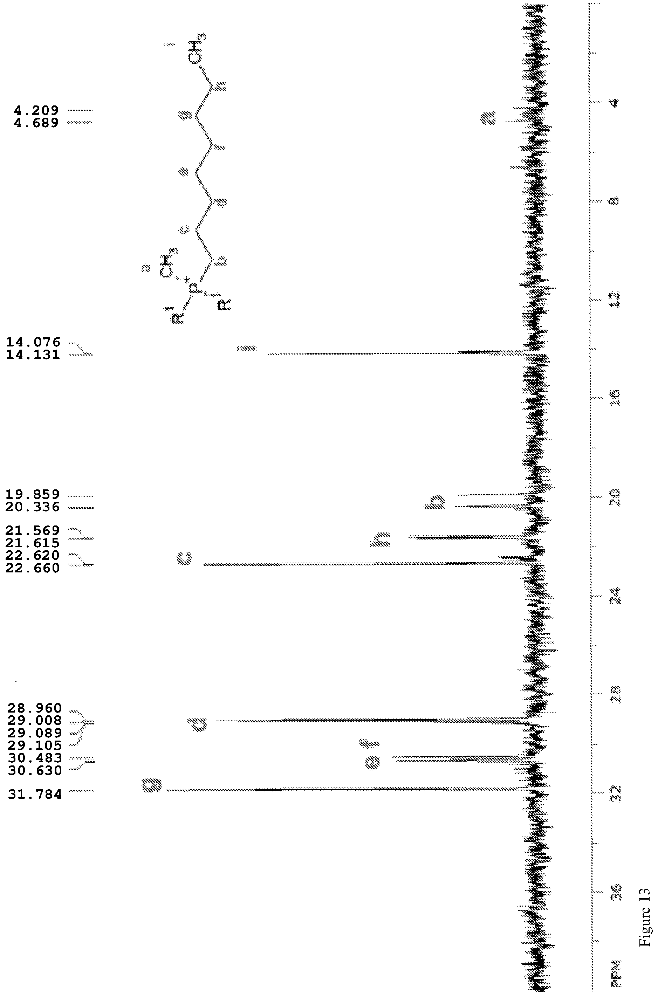

Figure 13 depicts the 13C NMR spectrum of the product of Example 2 ([P_{1,8,8,8}]₂ [Mo₂O₂S₆]) after further purification by solvent based separation.

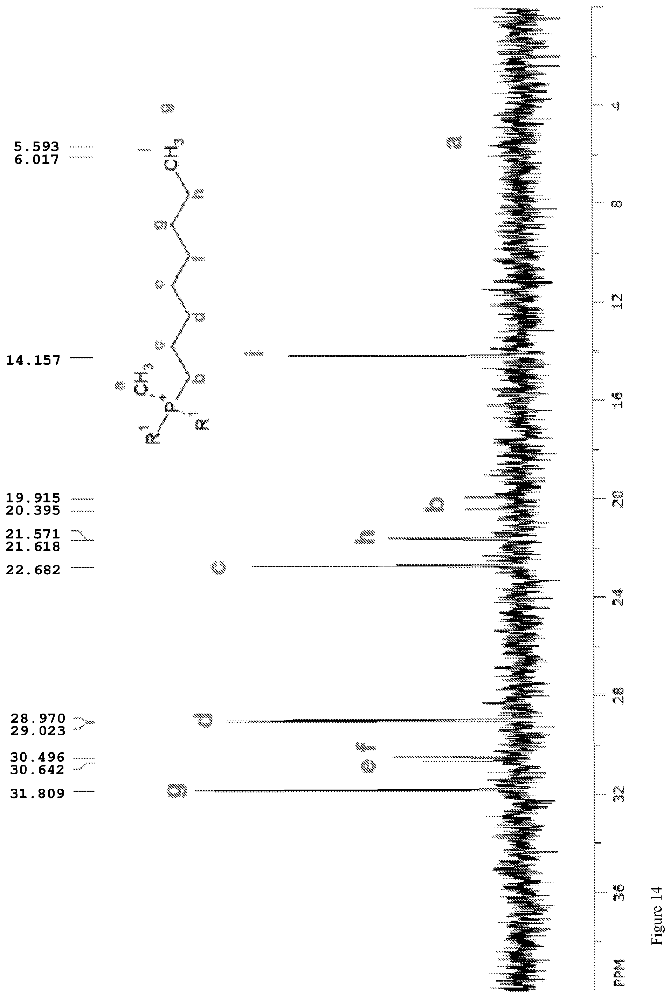

Figure 14 depicts the 13C NMR spectrum of the product of Example 3 ([P_{1,8,8,8}]₂ [Mo₂O₂S₆]).

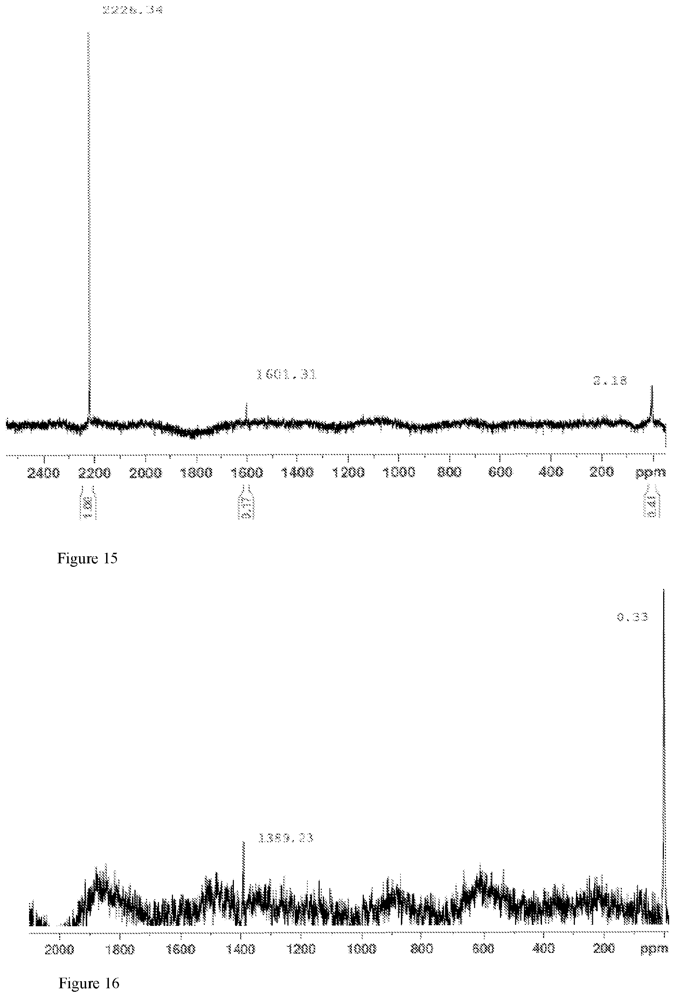

Figure 15 depicts the 95Mo NMR spectrum of the product of Example 1 ([P_{1,8,8,8}]₂ [MoS₄]).

Figure 16 depicts the 95Mo NMR spectrum of the product of Example 2 ([P_{1,8,8,8}]₂ [Mo₂O₂S₆]).

A comparison of the infra-red spectra show differences in the peaks for [P_{1,8,8,8}]₂[MoS₄] and [P_{1,8,8,8}]₂[Mo₂O₂S₆] between 400 and 600 cm⁻¹. The differences in peaks demonstrate different anionic environment before and after thermal treatment. The spectrum of [P_{1,8,8,8}]₂[MoS₂] produced by direct synthesis matches with the one obtained after thermal treatment.

The UV-Visible spectra for [P_{1,8,8,8}]₂[MoS₄] and [P_{1,8,8,8}]₂[Mo₂O₂S₆] formed by thermal treatment show differences corresponding to the difference in anionic environment. The spectrum of [P_{1,8,8,8}]₂[MoS₄] produced by direct synthesis matches with the one obtained after thermal treatment.

¹H-NMR spectra of the cation show changes on the methyl group shifting towards lower chemical shift after thermal treatment reflecting the interactions with different anionic species before and after thermal treatment. The spectrum of direct synthesis of [P_{1,8,8,8}]₂[Mo₂O₂S₆] matches with the one obtained after thermal treatment.

¹³C-NMR spectra of the cation show no changes upon thermal treatment demonstrating no change in the cation upon thermal treatment. The spectrum of direct synthesis of [P_{1,8,8,8}]₂[Mo₂O₂S₆] matches with the ones obtained before and after thermal treatment.

⁹⁵Mo-NMR peaks show differences before and after thermal treatment demonstrating the existence of the [MoS₄]²⁻ anion before thermal treatment and the [Mo₂O₂S₆]²⁻ anion after thermal treatment.

Table 1 shows the theoretically calculated percentage of Mo, P and S for the compounds [P_{1,8,8,8}]₂[Mo₂O₂S₆] and [P_{1,8,8,8}]₂[MoS₄]. Table 1 also shows the experimentally determined percentages for these elements for Example 1 ([P_{1,8,8,8}]₂[MoS₄]) and Example 2 ([P_{1,8,8,8}]₂[Mo₂O₂S₆]). Table 2 shows the theoretically calculated percentage of Mo, P and S for the compound ([P_{1,8,8,8}]₂[Mo₂O₂S₈]) and the experimentally determined percentages for these elements for Example 4 ([P_{1,8,8,8}]₂[Mo₂O₂S₈]).

**Table 1**

| % | Example 1 | Theoretical [P_{1,8,8,8}]₂[MoS₂] | Example 2 | Theoretical [P_{1,8,8,8}]₂[Mo₂O₂S₆] |
|---|---|---|---|---|
| Mo | 10.1 | 9.64 | 15.0 | 16.16 |
| P | 5.98 | 6.22 | 4.9 | 5.22 |
| S | 13.3 | 12.88 | 17.4 | 16.2 |

**Table 2**

| % | Example 4 | Theoretical [P_{1,8,8,8}]₂[Mo₂O₂S₈] |
|---|---|---|
| Mo | 14.2 | 15.3 |
| P | 5.3 | 5.0 |
| S | 20.4 | 20.5 |

The data in tables 1 and 2 shows that the elemental analysis of the products of Examples 1, 2 and 4 aligns with theoretical estimation of the elemental analysis of the anions [Mo₂O₂S₆]²⁻, [MoS₄]²⁻, and [Mo₂O₂S₈]²⁻.

### Tribological performance tests

Tribological performance of [P P_{1,8,8,8}]₂ [Mo₂O₂S₆] ionic liquids were tested in standard lubricant oil matrixes by measuring coefficient of friction and anti-wear properties using Schwingung, Reibung und Verschleiss (SRV) with the following conditions shown in Table 3.

**Table 3 - SRV conditions**

| | |
|---|---|
| Temperature (°C) | 100 |
| Frequency (Hz) | 10 |
| Stroke (mm) | 5 |
| Speed (mm/s) | Boundary |
| Load | 200 N |
| Pressure (GPa) | 2.7 |
| Type of Motion | Pure sliding |

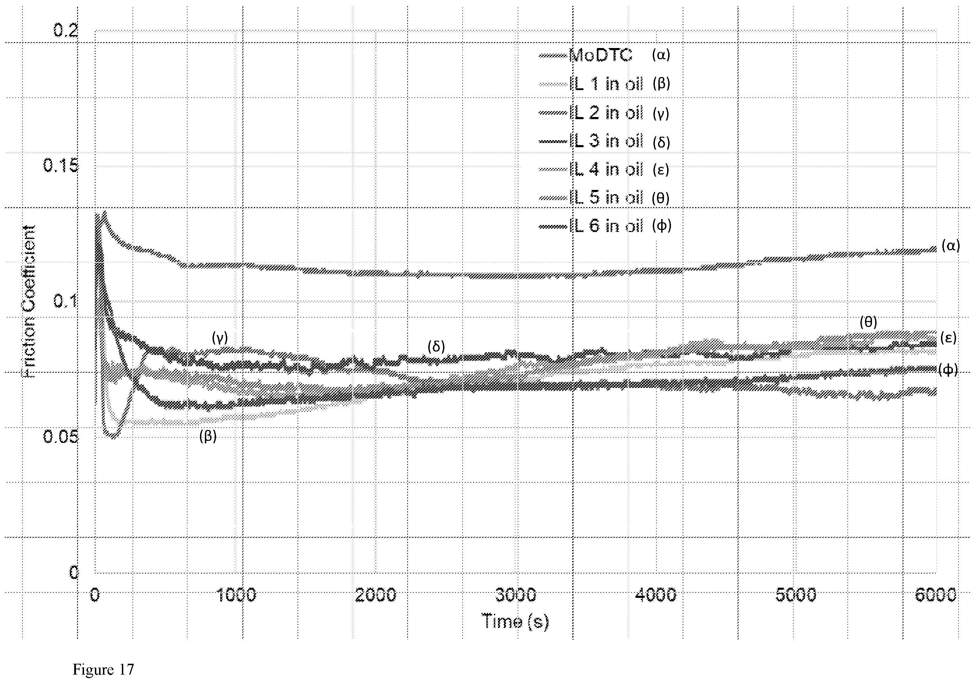

Figure 17 shows the coefficient of friction of the commercially available anti-friction additive MoDTC in a certain lubricating oil compared to the coefficient of friction of 6 batches of the ionic liquid [P_{1,8,8,8}]₂ [Mo₂O₂S₆] in the same lubricating oil. It can be seen that each oil batch containing the ionic liquid [P_{1,8,8,8}]₂ [Mo₂O₂S₆] had a lower coefficient of friction than the batch containing the additive MoDTC.

**Table 4 compares the friction reducing and anti-wear effects of standard lubricant formulated oils comprising the ionic liquids [P_{1,8,8,8}]₂ [Mo₂O₂S₆] and [P_{1,8,8,8}]₂[MoS₄].**

| Additive | wt% inclusion of additive in oil | Mo (ppm) | S (ppm) | P (ppm) | Wear scar (µm) | Friction coefficient |
|---|---|---|---|---|---|---|
| [P_{1,8,8,8}]₂[MoS₄] | 0.4 | 403 | 500 | 224 | 104 | 0.06 |
| [P_{1,8,8,8}]₂ [Mo₂O₂S₆] | 0.3 | 418 | 496 | 150 | 103 | 0.06 |

The data in Table 4 shows the oils comprising comparable amounts of the two ionic liquids had very similar wear scar and friction coefficient values, indicating similar anti-friction and anti-wear properties for the two ionic liquid additives.

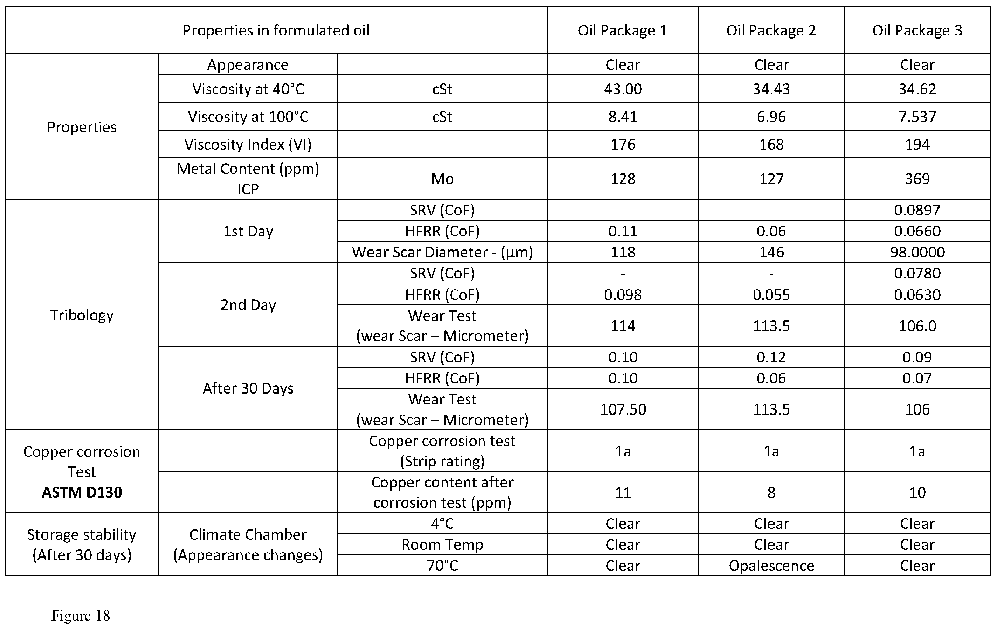

The data in Figure 18 shows the compatibility data for the ionic liquid [P_{1,8,8,8}]₂ [Mo₂O₂S₆] produced by thermal treatment (i.e. the product of Example 2) in various different lubricant oils. The data shows that the ionic liquid is stable and compatible for use with a variety of different oil packages.

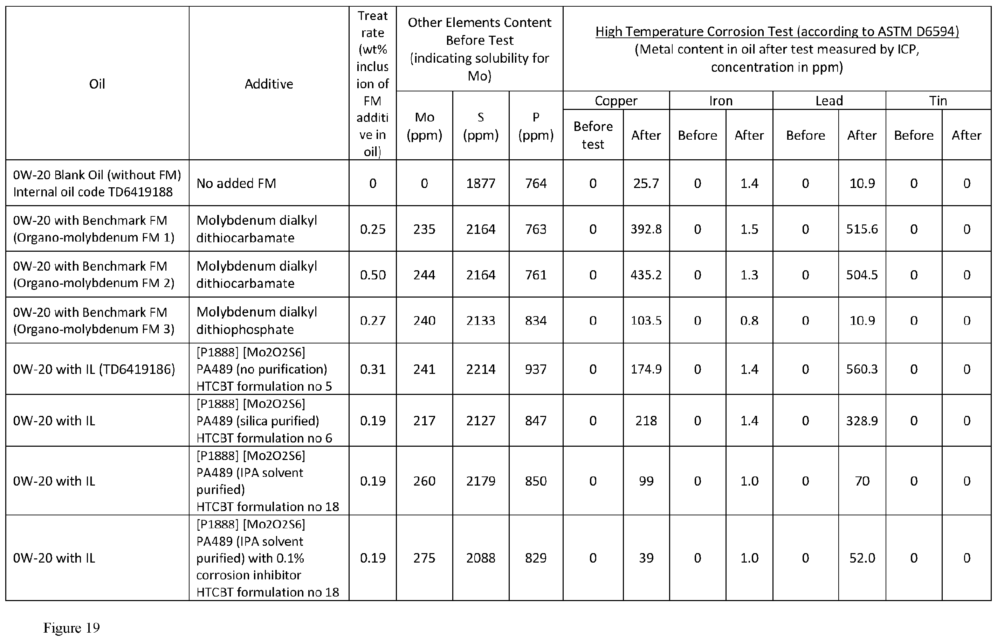

The data in Figure 19 shows the results of high temperature corrosion testing (according to ASTM D6594) for the product of Example 2 ([P_{1,8,8,8}]₂ [Mo₂O₂S₆]) where the ionic liquid is produced by thermal treatment and the commercially available additives molybdenum dialkyl dithiocarbamate and molybdenum dialkyl dithiophosphate. The data shows that the ionic liquid of Example 2, at similar treat rates, demonstrated reduced copper corrosion than molybdenum dialkyl dithiocarbamate and reduced copper corrosion compared to both molybdenum dialkyl dithiocarbamate and molybdenum dialkyl dithiophosphate after IPA solvent purification of the ionic liquid before use. The data also shows that after silica or IPA solvent purification, the ionic liquid demonstrates reduced lead corrosion relative to both molybdenum dialkyl dithiocarbamate and molybdenum dialkyl dithiophosphate

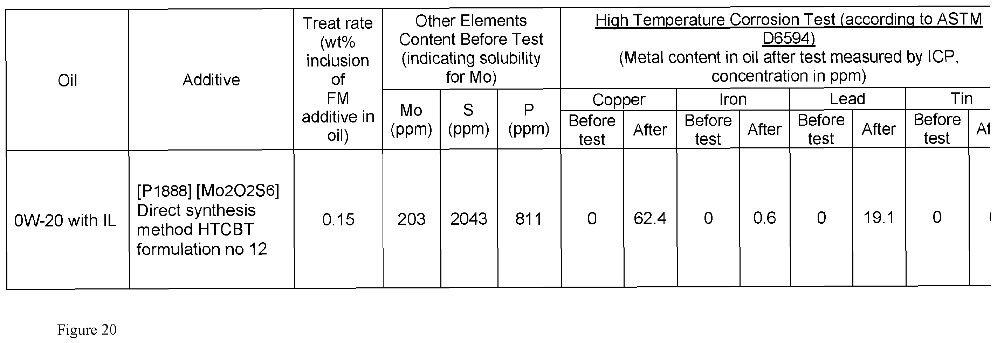

The data in Figure 20 shows the results of high temperature corrosion testing (according to ASTM D6594) for the product of Example 3 ([P_{1,8,8,8}]₂ [Mo₂O₂S₆]) where the ionic liquid is synthesised directly. The data shows even lower corrosion of both copper and lead than the ionic liquids and commercially available additives tested in the Figure 19 data.

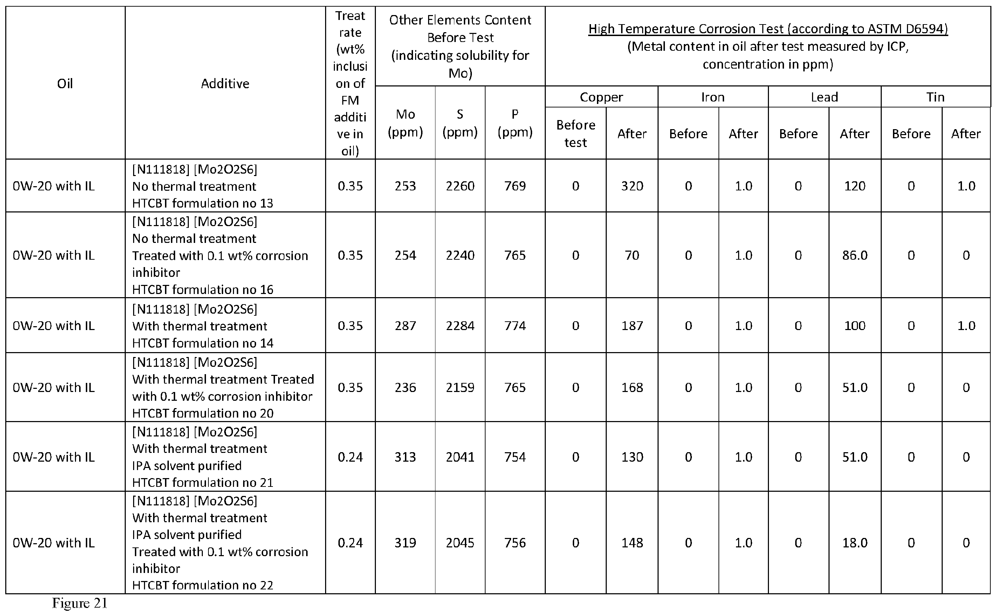

The data in Figure 21 shows the results of high temperature corrosion testing (according to ASTM D6594) for the ionic liquid [N_{1,1,18,18}][Mo₂O₂S₆]. The cation [N₁₁₁₈₁₈] is a dioctadecyldimethylammonium cation. The data shows that the ionic liquid has reduced copper and lead corrosion relative to the additive molybdenum dialkyl dithiocarbamate.

## Claims

1. A lubricant composition comprising one or more base oils and one or more ionic liquids, wherein the one or more ionic liquids comprise one or more cations and one or more binuclear molybdenum-containing metallate anions, wherein the one or more binuclear molybdenum-containing metallate anions is of the formula [Mo₂A_{b}C_{d}] and has a charge of -1 or -2, wherein A and C are each independently selected from O and S; and wherein b and d are each from 0 to 8; wherein the sum of b and d is 8.

2. A lubricant composition according to Claim 1, wherein the one or more binuclear molybdenum-containing metallate anions has a charge of -2; and/or
wherein each molybdenum atom is ligated to 5 atoms.

3. A lubricant composition according to any one of Claims 1 to 2, wherein the one or more binuclear molybdenum-containing metallate anions is of the formula: 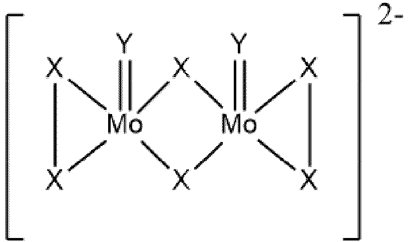 wherein each X and each Y is independently selected from O or S, preferably
wherein the one or more binuclear molybdenum-containing metallate anions comprises at least two O and/or at least two S atoms, or
wherein at least one X is O and at least one Y is S, or
wherein at least one X is S and at least one Y is O, preferably
wherein each X is S and at least one Y is O, and more preferably
wherein each X is S and each Y is O.

4. A lubricant composition according to any preceding claim, wherein the one or more ionic liquids have the formula: 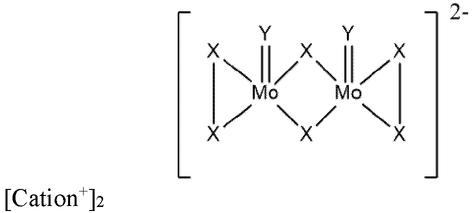 wherein X and Y are as defined in Claim 3; preferably wherein each X is S and each Y is O.

5. A lubricant composition according to any preceding claim, wherein the one or more cations comprise one or more cations selected from an imidazolium cation, a quaternary ammonium cation, a quaternary phosphonium cation, a sulphonium cation, or any combination thereof, preferably
wherein the one or more cations comprise one or more cations selected from the following structures: 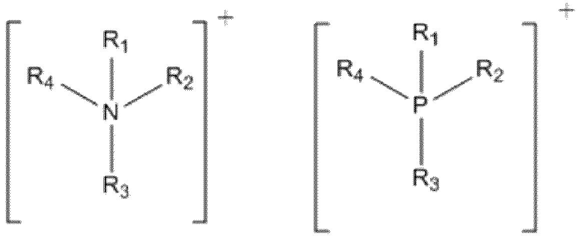 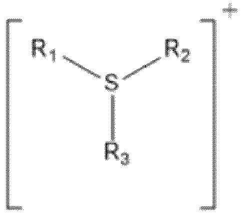 wherein R₁, R₂, R₃ and R₄ are independently selected from C₁ to C₃₀ straight chain or branched alkyl and alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}- wherein q is from 3 to 6; wherein any one or more of said straight or branched chain alkyl and alkenyl groups, cycloalkyl groups, arylalkyl groups, alkylaryl groups; aryl groups or alkylene chains are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl, more preferably
wherein R₁, R₂, R₃ and R₄ are C₁ to C₃₀ straight chain or branched alkyl and alkenyl groups, preferably C₁ to C₃₀ straight chain or branched alkyl groups, such as
wherein the one or more cations comprise one or more cations selected from the following structures: 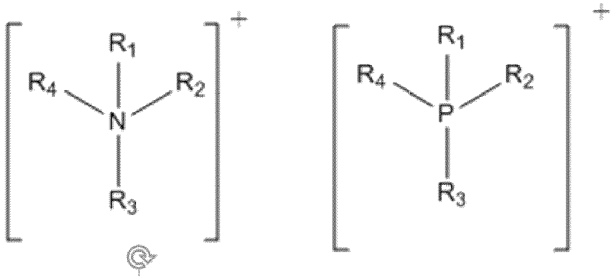
wherein R₁, R₂ and R₃ are the same and R₄ is different to R₁, R₂ and R₃; or wherein the one or more cations comprise one or more cations selected from the following structure: 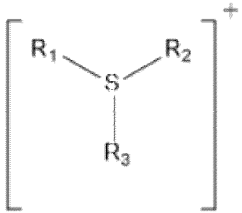
wherein R₁ and R₂ are the same and R₃ is different to R₁ and R₂, even more preferably R₁, R₂, R₃ and R₄ are C₁ to C₁₈ straight chain or branched alkyl groups, preferably C₁ to C₁₈ straight chain alkyl groups, and most preferably
wherein the one or more cations comprise one or more cations selected from the following structures: 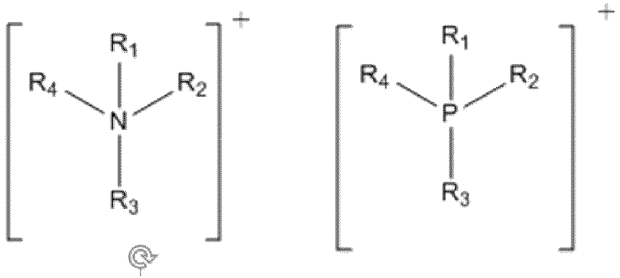 wherein R₁, R₂ and R₃ are C₄ to C₁₀ straight chain alkyl and wherein R₄ is C₁ to C₄ straight chain alkyl, for example
wherein R₁, R₂ and R₃ are octyl and wherein R₄ is methyl, and preferably
wherein the one or more cations comprise a phosphonium cation.

6. A lubricant composition according to Claim 5, wherein the one or more cations comprise one or more cations selected from the following structures: 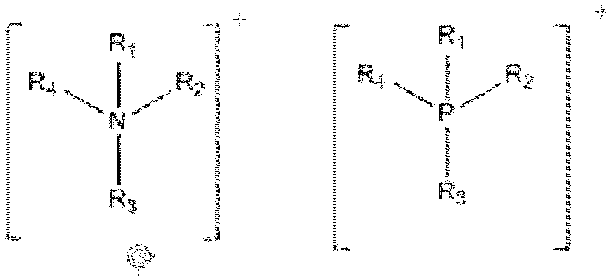 wherein R₁ and R₂ are C₁₄ to C₂₀ straight chain alkyl and wherein R₃ and R₄ are C₁ to C₄ straight chain alkyl, preferably
wherein R₁ and R₂ are octadecyl and wherein R₃ and R₄ are methyl, and more preferably wherein the one or more cations comprise an ammonium cation.

7. A lubricant composition according to any preceding claim, wherein the ionic liquid comprises [P_{1,8,8,8}]₂ [Mo₂O₂S₆] or [N_{1,1,18,18}] [Mo₂O₂S₆].

8. A lubricant composition according to any one of Claims 1 to 5, wherein the one or more cations comprise a substituted quaternary imidazolium cation comprising at least one ester group, or one or more cations selected from the following structures: 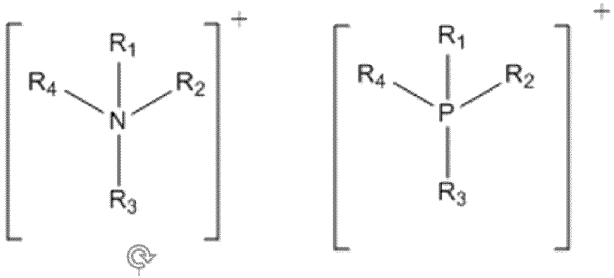 wherein at least one of R₁, R₂, R₃ and R₄ have the formula -R₅O(C=O)R₆ or -R₅(C=O)-O-R₆, wherein R₅ is a C₁ to C₁₀ straight chain or branched alkyl or alkenyl group, or a C₃ to C₆ cycloalkyl or cycloalkenyl group; and R₆ is a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and - OC(O)(C₁ to C₆)alkyl;
and wherein the other of R₁, R₂, R₃ and R₄ that are not as defined above are independently selected from C₁ to C₃₀ straight chain or branched alkyl and alkenyl groups such as C₁ to C₁₀ alkyl or alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}-wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl; or wherein the one or more cations comprise one or more cations selected from the following structure: 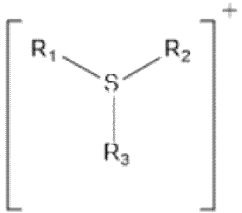 wherein at least one of R₁, R₂ and R₃ have the formula -R₅O(C=O)R₆ or -R₅(C=O)-O-R₆, wherein R₅ is a C₁ to C₁₀ straight chain or branched alkyl or alkenyl group, or a C₃ to C₆ cycloalkyl or cycloalkenyl group; and R₆ is a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and - OC(O)(C₁ to C₆)alkyl;
and wherein the other of R₁, R₂ and R₃ that are not as defined above are independently selected from C₁ to C₃₀ straight chain or branched alkyl and alkenyl groups such as C₁ to C₁₀ alkyl or alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}-wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl.

9. A lubricant composition according to any one of Claims 1 to 5, wherein the one or more cations comprise a substituted quaternary imidazolium cation comprising at least one amide group, or one or more cations selected from the following structures: 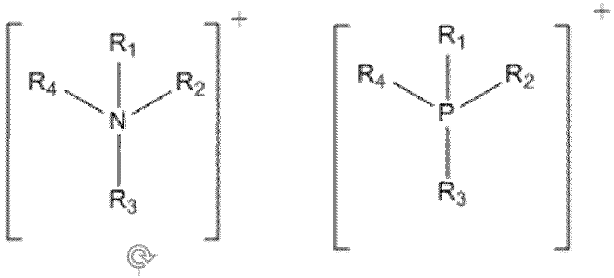 wherein at least one of R₁, R₂, R₃ and R₄ have the formula -R₅-N(R₇)-(C=O)R₆ or -R₅(C=O)-N(R₇)-R₆, wherein R₅ and R₆ are as defined in claim 23, and wherein R₇ is selected from H; a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl;
and wherein the other of R₁, R₂, R₃ and R₄ that are not as defined above are independently selected from C₁ to C₃₀ straight chain or branched alkyl and alkenyl groups such as C₁ to C₁₀ alkyl or alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}-wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl; or wherein the one or more cations comprise one or more cations selected from the following structure: 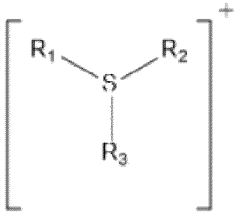 wherein at least one of R₁, R_{2,} and R₃ have the formula -R₅-N(R₇)-(C=O)R₆ or -R₅(C=O)-N(R₇)-R₆, wherein R₅ and R₆ are as defined in claim 23, and wherein R₇ is selected from H; a C₁ to C₃₀ straight chain or branched alkyl or alkenyl group; a C₃ to C₆ cycloalkyl group; a C₁ to C₃₀ arylalkyl group; a C₁ to C₃₀ alkylaryl group; an aryl group; or any two respective R₆ groups combine to form an alkylene chain -(CH₂)_{q}- wherein q is from 3 to 6; wherein R₅ and/or R₆ are optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl;
and wherein the other of R₁, R₂ and R₃ that are not as defined above are independently selected from C₁ to C₃₀ straight chain or branched alkyl and alkenyl groups such as C₁ to C₁₀ alkyl or alkenyl groups; C₃ to C₆ cycloalkyl groups; C₁ to C₃₀ arylalkyl groups; C₁ to C₃₀ alkylaryl groups; aryl groups; or any two of R₁, R₂, R₃ and R₄ combine to form an alkylene chain -(CH₂)_{q}-wherein q is from 3 to 6; wherein any one or more of said straight or branched chain alkyl and alkenyl groups, cycloalkyl groups, arylalkyl groups, alkylaryl groups; aryl groups or alkylene chains is optionally substituted by one to three groups selected from: C₁ to C₄ alkoxy, C₂ to C₈ alkoxyalkoxy, C₃ to C₆ cycloalkyl, -OH, -NH₂, -SH, -CO₂(C₁ to C₆)alkyl, and -OC(O)(C₁ to C₆)alkyl, preferably
wherein the one or more cations comprise one or more cations selected from the following structures: 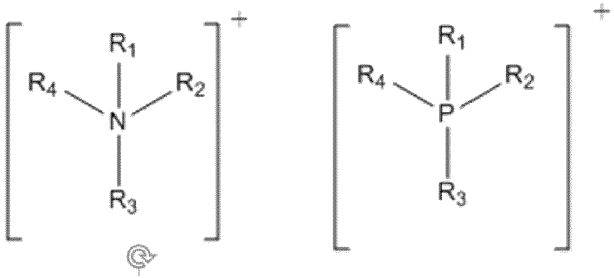 wherein three of R₁ R₂, R₃ and R₄ have the formula -R₅O(C=O)R₆; -R₅(C=O)-O-R₆; -R₅-N(R₇)-(C=O)R₆; or -R₅(C=O)-N(R₇)-R₆; wherein R₅, R₆ and R₇ are as defined above, more preferably
wherein three of R₁ R₂, R₃ and R₄ have the formula -R₅O(C=O)R₆, more preferably
wherein the cation comprises a quaternary ammonium cation or a quaternary phosphonium cation, such as
wherein the cation comprises a quaternary ammonium cation; R₁ R₂, and R₃ have the formula - R₅O(C=O)R₆ wherein R₅ is C₂H₄; and wherein R₄ is C₁ to C₅ alkyl, preferably methyl.

10. A lubricant composition according to any preceding claim, wherein the ionic liquid is a liquid at a temperature of 100°C and above, preferably 50°C and above, and/or
wherein the ionic liquid is a liquid at a temperature of 25°C and above, preferably 20°C and above, more preferably 10°C and above, and most preferably 0°C and above.

11. A lubricant composition according to any preceding claim, wherein the ionic liquid comprises less than 5 wt% halide, preferably less than 2.5 wt. % halide, and most preferably less than 1 wt. % halide, preferably
wherein the ionic liquid comprises less than 0.5 wt% halide, and preferably less than 0.1 wt. % halide, and more preferably
wherein the ionic liquid is free of halide.

12. A lubricant composition according to any preceding claim, wherein the composition comprises the one or more ionic liquids in an amount of from 0.05 wt.% to 20 wt. %, preferably from 0.1 wt. % to 10 wt. %, and most preferably from 0.1 wt. % to 5 wt. %, such as wherein the composition comprises the one or more ionic liquids in an amount of from 0.5 wt. % to 1.5 wt. %.

13. A lubricant composition according to any preceding claim, wherein the one or more base oils are present in the composition in an amount of from 75% to 99% by weight of the lubricant composition.

14. A lubricant composition according to any preceding claim, wherein the one or more base oils comprise one or more base stocks selected from Group I base stocks, Group II base stocks, Group III base stocks, Group IV base stocks and Group V base stocks, and/or wherein the one or more base oils comprise one or more synthetic oils selected from polyalphaolefins, synthetic esters, polyalkylene glycols, phosphate esters, alkylated naphthalenes, silicate esters, ionic fluids, or multiply alkylated cyclopentanes.

15. A lubricant composition according to any preceding claim, further comprising one or more additives optionally selected from one or more pour point additives, anti-foaming additives, viscosity index improvers, antioxidants, detergents, corrosion inhibitors, anti-wear additives, friction modifiers, extreme pressure additives, or any combination thereof.

16. A lubricant composition according to any preceding claim, wherein the composition is a lubricating oil composition, such as
wherein the composition is a grease composition; optionally, wherein the grease composition comprises from 50% to 95% by weight of one or more base oils; and from 5% to 50% by weight of one or more thickeners; preferably wherein the grease composition comprises from 10% to 30% by weight of one or more thickeners, preferably
wherein the one or more thickeners comprise soap such as an alkali metal or alkaline earth metal salt of a C₁₄ to C₂₀ fatty acid, or derivatives thereof; an alkaline earth metal sulphonate such as calcium sulphonate; a urea; a substituted urea; a polyurea; a substituted polyurea; clay; tar; graphite; mica; silica; or a combination thereof.

17. A method of producing a lubricant composition according to any preceding claim, comprising the steps of combining one or more base oils with one or more ionic liquids, and optionally one or more additives or one or more thickeners.

18. A method of operating industrial machinery, a spark ignition engine or a compression ignition engine comprising lubricating said engine with a lubricant composition according to any preceding claim.

19. An ionic liquid comprising one or more cations and one or more binuclear molybdenum-containing metallate anions, wherein the one or more binuclear molybdenum-containing metallate anions is of the formula [Mo₂A_{b}C_{d}] and has a charge of -1 or -2, wherein A and C are each independently selected from O and S; and wherein b and d are each from 0 to 8; wherein the sum of b and d is 8, preferably
wherein the ionic liquid is as defined in any one or more of Claims 2 to 11.

20. Use of an ionic liquid according to Claim 19, in a lubricant composition, for the purpose of improving the anti-friction properties of the lubricant composition and/or for the purpose of improving the anti-wear properties of the lubricant composition, preferably
wherein the lubricant composition is as defined in any one of claims 1 to 16, more preferably
wherein the ionic liquid is used for the purpose of improving the anti-friction properties of the lubricant composition, or
wherein the ionic liquid is used for the purpose of improving the anti-wear properties of the lubricant composition.

21. An additive concentrate for use in a lubricant composition, wherein the additive concentrate comprises an ionic liquid according to Claim 19, or as defined in any one of claims 1 to 11, and one or more further additives selected from one or more pour point additives, anti-foaming additives, viscosity index improvers, antioxidants, detergents, corrosion inhibitors, anti-wear additives, friction modifiers, extreme pressure additives, or any combination thereof.

22. A process for making an ionic liquid according to Claim 19, wherein the process comprises:
(a) providing an ionic liquid comprising one or more cations as defined in any one of Claims 5 to 9 and one or more anions of the following formula: 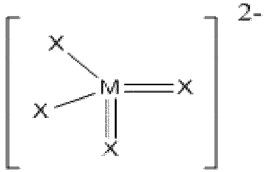 wherein each X is independently selected from O or S; and wherein M = Mo; and
(b) heating the ionic liquid from step (a) to a temperature of from 70°C to 130°C in the presence of air or oxygen so as to provide the ionic liquid according to Claim 19; preferably wherein the ionic liquid is heated to a temperature of from 80°C to 120°C, more preferably wherein step (b) further comprises stirring the ionic liquid from step (a), and/or wherein the one or more anions of the ionic liquid in step (a) are of the following formula: 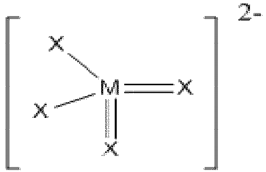 wherein each X is O; or wherein each X is S; preferably wherein each X is S.

23. A process for making an ionic liquid according to Claim 19, wherein the process comprises:
(a) forming a solution of a salt comprising one or more cations and one or more anions of the following formula: 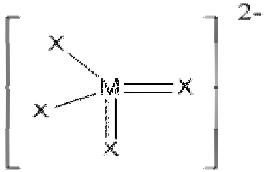 wherein each X is independently selected from O or S; and wherein M = Mo;
(b) heating the solution from step (a) to a temperature of from 70°C to 130°C in the presence of air or oxygen; preferably wherein the solution is heated to a temperature of from 80°C to 120°C;
(c) optionally, adding one or more bases to the solution; and
(d) contacting the solution with a salt comprising one or more cations as defined in any one of Claims 5 to 9 and one or more anions to form the ionic liquid according to Claim 19, preferably wherein step (a) comprises stirring the salt in a suitable solvent to form the solution, and/or wherein step (d) further comprises stirring the solution upon contacting, and/or wherein the salt added to the solution in step (d) is in the form of a solution.

## Patentansprüche

1. Eine Schmiermittelzusammensetzung, die ein oder mehrere Basisöle und eine oder mehrere ionische Flüssigkeiten umfasst, wobei die eine oder mehreren ionischen Flüssigkeiten ein oder mehrere Kationen und ein oder mehrere zweikernige molybdänhaltige Metallatanionen umfassen, wobei das eine oder die mehreren zweikernigen molybdänhaltigen Metallatanionen die Formel [Mo₂A_{b}C_{d}] aufweisen und eine Ladung von -1 oder -2 haben, wobei A und C jeweils unabhängig aus O und S ausgewählt sind; und wobei b und d jeweils von 0 bis 8 sind; wobei die Summe von b und d 8 ist.

2. Schmiermittelzusammensetzung nach Anspruch 1, wobei das eine oder die mehreren zweikernigen molybdänhaltigen Metallatanionen eine Ladung von -2 haben; und/oder wobei jedes Molybdänatom an 5 Atome ligiert ist.

3. Schmiermittelzusammensetzung nach einem der Ansprüche 1 bis 2, wobei das eine oder die mehreren zweikernigen molybdänhaltigen Metallatanionen die folgende Formel aufweisen: 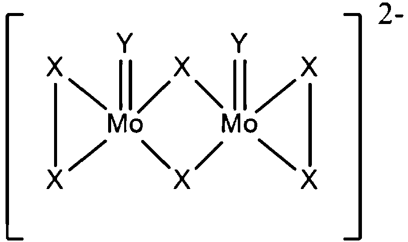 wobei jedes X und jedes Y unabhängig aus O oder S ausgewählt ist, bevorzugt
wobei das eine oder die mehreren zweikernigen molybdänhaltigen Metallatanionen mindestens zwei O und/oder mindestens zwei S-Atome umfassen oder
wobei mindestens ein X O ist und mindestens ein Y S ist oder
wobei mindestens ein X S ist und mindestens ein Y O ist, bevorzugt
wobei jedes X S ist und mindestens ein Y O ist und bevorzugter
wobei jedes X S ist und jedes Y O ist.

4. Schmiermittelzusammensetzung nach einem der vorhergehenden Ansprüche, wobei die eine oder mehreren ionischen Flüssigkeiten die folgende Formel aufweisen: 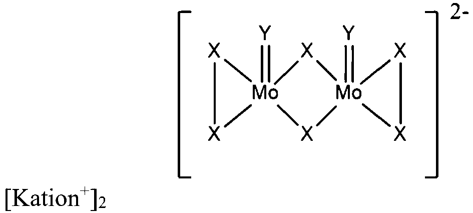 wobei X und Y wie in Anspruch 3 definiert sind; wobei bevorzugt jedes X S ist und jedes Y O ist.

5. Schmiermittelzusammensetzung nach einem der vorhergehenden Ansprüche, wobei das eine oder die mehreren Kationen ein oder mehrere Kationen umfassen, die aus einem Imidazoliumkation, einem quartären Ammoniumkation, einem quartären Phosphoniumkation, einem Sulfoniumkation oder einer beliebigen Kombination davon ausgewählt sind, bevorzugt
wobei das eine oder die mehreren Kationen ein oder mehrere Kationen umfassen, die aus den folgenden Strukturen ausgewählt sind: 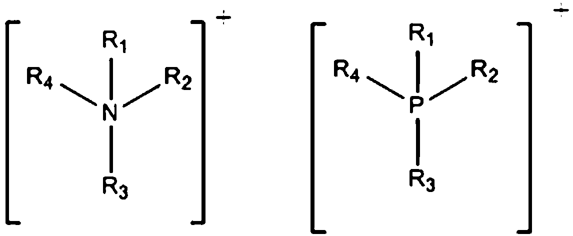 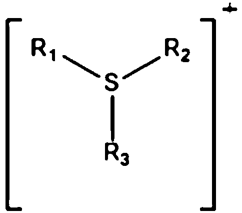 wobei R₁, R₂, R₃ und R₄ unabhängig aus geradkettigen oder verzweigten C₁- bis C₃₀-Alkyl- und Alkenylgruppen; C₃- bis C₆-Cycloalkylgruppen; C₁- bis C₃₀-Arylalkylgruppen; C₁- bis C₃₀-Alkylarylgruppen; Arylgruppen ausgewählt sind; oder beliebige zwei von R₁, R₂, R₃ und R₄ sich vereinigen, um eine Alkylenkette -(CH₂)_{q}- zu bilden, wobei q von 3 bis 6 ist; wobei beliebige eine oder mehrere der genannten gerad- oder verzweigtkettigen Alkyl- und Alkenylgruppen, Cycloalkylgruppen, Arylalkylgruppen, Alkylarylgruppen; Arylgruppen oder Alkylenketten optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁-bis C₆)alkyl, bevorzugter
wobei R₁, R₂, R₃ und R₄ geradkettige oder verzweigte C₁- bis C₃₀-Alkyl- und Alkenylgruppen, bevorzugt geradkettige oder verzweigte C₁- bis C₃₀-Alkylgruppen, sind, wie etwa
wobei das eine oder die mehreren Kationen ein oder mehrere Kationen umfassen, die aus den folgenden Strukturen ausgewählt sind: 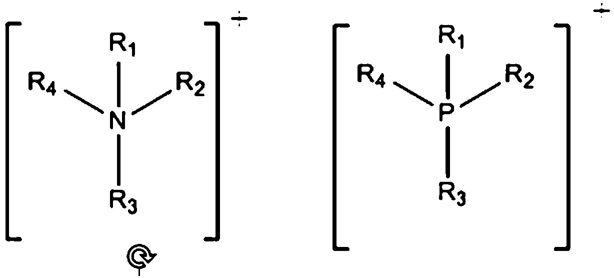
wobei R₁, R₂ und R₃ gleich sind und R₄ verschieden von R₁, R₂ und R₃ ist; oder wobei das eine oder die mehreren Kationen ein oder mehrere Kationen umfassen, die aus der folgenden Struktur ausgewählt sind: 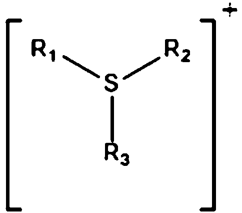 wobei R₁ und R₂ gleich sind und R₃ verschieden von R₁ und R₂ ist, noch bevorzugter
R₁, R₂, R₃ und R₄ geradkettige oder verzweigte C₁- bis C₁₈ -Alkylgruppen, bevorzugt geradkettige C₁- bis C₁₈-Alkylgruppen, sind und am bevorzugtesten
wobei das eine oder die mehreren Kationen ein oder mehrere Kationen umfassen, die aus den folgenden Strukturen ausgewählt sind: 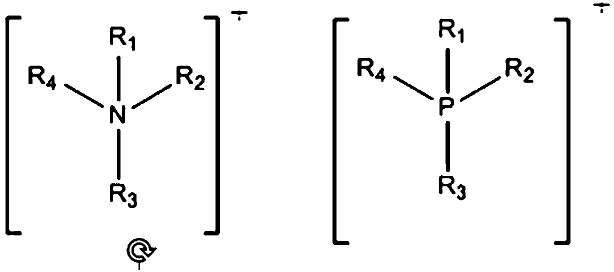 wobei R₁, R₂ und R₃ geradkettiges C₄- bis C₁₀-Alkyl sind und wobei R₄ geradkettiges C₁- bis C₄-Alkyl ist, zum Beispiel
wobei R₁, R₂ und R₃ Octyl sind und wobei R₄ Methyl ist, und bevorzugt
wobei das eine oder die mehreren Kationen ein Phosphoniumkation umfassen.

6. Schmiermittelzusammensetzung nach Anspruch 5, wobei das eine oder die mehreren Kationen ein oder mehrere Kationen umfassen, die aus den folgenden Strukturen ausgewählt sind: 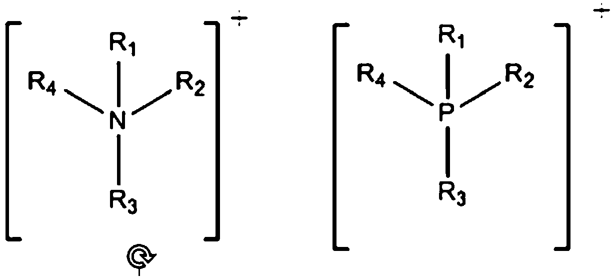 wobei R₁ und R₂ geradkettiges C₁₄- bis C₂₀-Alkyl sind und wobei R₃ und R₄ geradkettiges C₁- bis C₄-Alkyl sind, bevorzugt
wobei R₁ und R₂ Octadecyl sind und wobei R₃ und R₄ Methyl sind, und bevorzugter
wobei das eine oder die mehreren Kationen ein Ammoniumkation umfassen.

7. Schmiermittelzusammensetzung nach einem der vorhergehenden Ansprüche, wobei die ionische Flüssigkeit [P_{1,8,8,8}]₂ [Mo₂O₂S₆] oder [N_{1,1,18,18}] [Mo₂O₂S₆] umfasst.

8. Schmiermittelzusammensetzung nach einem der Ansprüche 1 bis 5, wobei das eine oder die mehreren Kationen ein substituiertes quartäres Imidazoliumkation, das mindestens eine Estergruppe umfasst, oder ein oder mehrere Kationen umfassen, die aus den folgenden Strukturen ausgewählt sind: 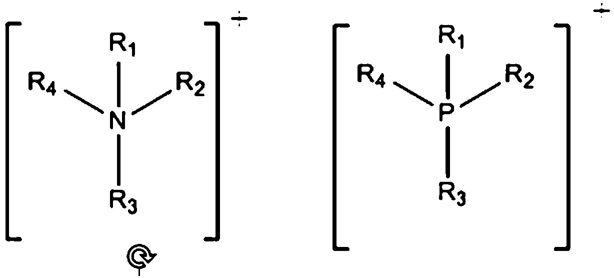 wobei mindestens eines von R₁, R₂, R₃ und R₄ die Formel -R₅O(C=O)R₆ oder -R₅(C=O)-O-R₆ aufweist, wobei R₅ eine geradkettige oder verzweigte C₁- bis C₁₀-Alkyl- oder Alkenylgruppe oder eine C₃- bis C₆-Cycloalkyl- oder Cycloalkenylgruppe ist; und R₆ eine geradkettige oder verzweigte C₁- bis C₃₀-Alkyl- oder Alkenylgruppe; eine C₃- bis C₆-Cycloalkylgruppe; eine C₁- bis C₃₀-Arylalkylgruppe; eine C₁- bis C₃₀-Alkylarylgruppe; eine Arylgruppe ist; oder zwei beliebige jeweilige R₆-Gruppen sich vereinigen, um eine Alkylenkette -(CH₂)_{q}- zu bilden, wobei q von 3 bis 6 ist; wobei R₅ und/oder R₆ optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁-bis C₆)alkyl und -OC(O)(C₁- bis C₆)alkyl;
und wobei die anderen von R₁, R₂, R₃ und R₄, die nicht wie oben definiert sind, unabhängig aus geradkettigen oder verzweigten C₁- bis C₃₀-Alkyl- und Alkenylgruppen, wie etwa C₁- bis C₁₀-Alkyl- oder Alkenylgruppen; C₃- bis C₆-Cycloalkylgruppen; C₁- bis C₃₀-Arylalkylgruppen; C₁- bis C₃₀-Alkylarylgruppen; Arylgruppen ausgewählt sind; oder beliebige zwei von R₁, R₂, R₃ und R₄ sich vereinigen, um eine Alkylenkette -(CH₂)_{q}- zu bilden, wobei q von 3 bis 6 ist; wobei beliebige eine oder mehrere der genannten gerad- oder verzweigtkettigen Alkyl- und Alkenylgruppen, Cycloalkylgruppen, Arylalkylgruppen, Alkylarylgruppen; Arylgruppen oder Alkylenketten optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁-bis C₆)alkyl; oder wobei das eine oder die mehreren Kationen ein oder mehrere Kationen umfassen, die aus der folgenden Struktur ausgewählt sind: 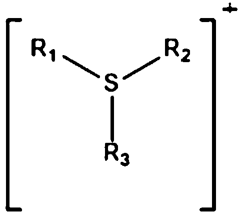 wobei mindestens eines von R₁, R₂ und R₃ die Formel -R₅O(C=O)R₆ oder -R₅(C=O)-O-R₆ aufweist, wobei R₅ eine geradkettige oder verzweigte C₁- bis C₁₀-Alkyl- oder Alkenylgruppe oder eine C₃- bis C₆-Cycloalkyl- oder Cycloalkenylgruppe ist; und R₆ eine geradkettige oder verzweigte C₁- bis C₃₀-Alkyl- oder Alkenylgruppe; eine C₃- bis C₆-Cycloalkylgruppe; eine C₁- bis C₃₀-Arylalkylgruppe; eine C₁- bis C₃₀-Alkylarylgruppe; eine Arylgruppe ist; oder zwei beliebige jeweilige R₆-Gruppen sich vereinigen, um eine Alkylenkette -(CH₂)_{q}- zu bilden, wobei q von 3 bis 6 ist; wobei R₅ und/oder R₆ optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁-bis C₆)alkyl und -OC(O)(C₁- bis C₆)alkyl;
und wobei die anderen von R₁, R₂ und R₃, die nicht wie oben definiert sind, unabhängig aus geradkettigen oder verzweigten C₁- bis C₃₀-Alkyl- und Alkenylgruppen, wie etwa C₁- bis C₁₀-Alkyl- oder Alkenylgruppen; C₃- bis C₆-Cycloalkylgruppen; C₁- bis C₃₀-Arylalkylgruppen; C₁- bis C₃₀-Alkylarylgruppen; Arylgruppen ausgewählt sind; oder beliebige zwei von R₁, R₂, R₃ und R₄ sich vereinigen, um eine Alkylenkette -(CH₂)_{q}- zu bilden, wobei q von 3 bis 6 ist; wobei beliebige eine oder mehrere der genannten gerad- oder verzweigtkettigen Alkyl- und Alkenylgruppen, Cycloalkylgruppen, Arylalkylgruppen, Alkylarylgruppen; Arylgruppen oder Alkylenketten optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁-bis C₆)alkyl.

9. Schmiermittelzusammensetzung nach einem der Ansprüche 1 bis 5, wobei das eine oder die mehreren Kationen ein substituiertes quartäres Imidazoliumkation, das mindestens eine Amidgruppe umfasst, oder ein oder mehrere Kationen umfassen, die aus den folgenden Strukturen ausgewählt sind: 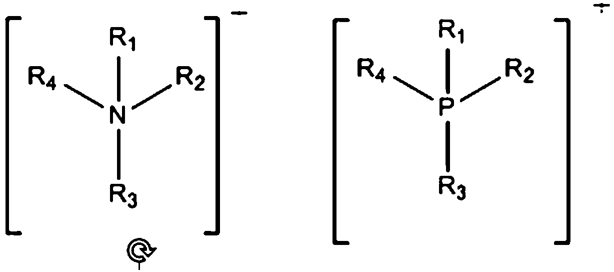 wobei mindestens eines von R₁, R₂, R₃ und R₄ die Formel -R₅-N(R₇)-(C=O)R₆ oder -R₅(C=O)-N(R₇)-R₆ aufweist, wobei R₅ und R₆ wie in Anspruch 23 definiert sind, und wobei R₇ aus H; einer geradkettigen oder verzweigten C₁- bis C₃₀-Akyl- oder Alkenylgruppe; einer C₃- bis C₆-Cycloalkylgruppe; einer C₁- bis C₃₀-Arylalkylgruppe; einer C₁- bis C₃₀-Alkylarylgruppe; einer Arylgruppe ausgewählt ist; oder zwei beliebige jeweilige R₆-Gruppen sich vereinigen, um eine Alkylenkette -(CH₂)_{q}- zu bilden, wobei q von 3 bis 6 ist; wobei R₅ und/oder R₆ optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁- bis C₆)alkyl;
und wobei die anderen von R₁, R₂, R₃ und R₄, die nicht wie oben definiert sind, unabhängig aus geradkettigen oder verzweigten C₁- bis C₃₀-Alkyl- und Alkenylgruppen, wie etwa C₁- bis C₁₀-Alkyl- oder Alkenylgruppen; C₃- bis C₆-Cycloalkylgruppen; C₁- bis C₃₀-Arylalkylgruppen; C₁- bis C₃₀-Alkylarylgruppen; Arylgruppen ausgewählt sind; oder beliebige zwei von R₁, R₂, R₃ und R₄ sich vereinigen, um eine Alkylenkette -(CH₂)_{q}- zu bilden, wobei q von 3 bis 6 ist; wobei beliebige eine oder mehrere der genannten gerad- oder verzweigtkettigen Alkyl- und Alkenylgruppen, Cycloalkylgruppen, Arylalkylgruppen, Alkylarylgruppen; Arylgruppen oder Alkylenketten optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁-bis C₆)alkyl; oder wobei das eine oder die mehreren Kationen ein oder mehrere Kationen umfassen, die aus der folgenden Struktur ausgewählt sind: 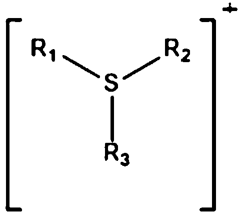 wobei mindestens eines von R₁, R₂ und R₃ die Formel -R₅-N(R₇)-(C=O)R₆ oder -R₅(C=O)-N(R₇)-R₆ aufweist, wobei R₅ und R₆ wie in Anspruch 23 definiert sind und wobei R₇ aus H; einer geradkettigen oder verzweigten C₁- bis C₃₀-Alkyl- oder Alkenylgruppe; einer C₃- bis C₆-Cycloalkylgruppe; einer C₁- bis C₃₀-Arylalkylgruppe; einer C₁- bis C₃₀-Alkylarylgruppe; einer Arylgruppe ausgewählt ist; oder zwei beliebige jeweilige R₆-Gruppen sich vereinigen, um eine Alkylenkette -(CH₂)_{q}- zu bilden, wobei q von 3 bis 6 ist; wobei R₅ und/oder R₆ optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁- bis C₆)alkyl;
und wobei die anderen von R₁, R₂ und R₃, die nicht wie oben definiert sind, unabhängig aus geradkettigen oder verzweigten C₁- bis C₃₀-Alkyl- und Alkenylgruppen, wie etwa C₁- bis C₁₀-Alkyl- oder Alkenylgruppen; C₃- bis C₆-Cycloalkylgruppen; C₁- bis C₃₀-Arylalkylgruppen; C₁- bis C₃₀-Alkylarylgruppen; Arylgruppen ausgewählt sind; oder beliebige zwei von R₁, R₂, R₃ und R₄ sich vereinigen, um eine Alkylenkette -(CH₂)_{q}- zu bilden, wobei q von 3 bis 6 ist; wobei beliebige eine oder mehrere der genannten gerad- oder verzweigtkettigen Alkyl- und Alkenylgruppen, Cycloalkylgruppen, Arylalkylgruppen, Alkylarylgruppen; Arylgruppen oder Alkylenketten optional durch eine bis drei Gruppen substituiert sind, ausgewählt aus: C₁- bis C₄-Alkoxy, C₂- bis C₈-Alkoxyalkoxy, C₃- bis C₆-Cycloalkyl, -OH, -NH₂, -SH, -CO₂(C₁- bis C₆)alkyl und -OC(O)(C₁-bis C₆)alkyl, bevorzugt
wobei das eine oder die mehreren Kationen ein oder mehrere Kationen umfassen, die aus den folgenden Strukturen ausgewählt sind: 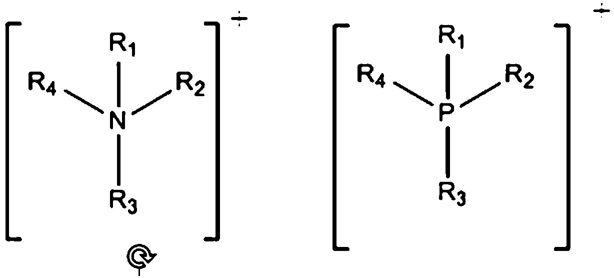 wobei drei von R₁ R₂, R₃ und R₄ die Formel -R₅O(C=O)R₆; -R₅(C=O)-O-R₆; -R₅-N(R₇)-(C=O)R₆ oder -R₅(C=O)-N(R₇)-R₆ aufweisen; wobei R₅, R₆ und R₇ wie oben definiert sind, bevorzugter
wobei drei von R₁ R₂, R₃ und R₄ die Formel -R₅O(C=O)R₆ aufweisen, bevorzugter
wobei das Kation ein quartäres Ammoniumkation oder ein quartäres Phosphoniumkation umfasst, wie etwa
wobei das Kation ein quartäres Ammoniumkation umfasst; R₁, R₂ und R₃ die Formel - R₅O(C=O)R₆ aufweisen, wobei R₅ C₂H₄ ist; und wobei R₄ C₁- bis C₅-Alkyl, bevorzugt Methyl, ist.

10. Schmiermittelzusammensetzung nach einem der vorhergehenden Ansprüche, wobei die ionische Flüssigkeit eine Flüssigkeit mit einer Temperatur von 100°C und darüber, bevorzugt 50°C und darüber, ist, und/oder
wobei die ionische Flüssigkeit eine Flüssigkeit mit einer Temperatur von 25°C und darüber, bevorzugt 20°C und darüber, bevorzugter 10°C und darüber und am bevorzugtesten 0°C und darüber, ist.

11. Schmiermittelzusammensetzung nach einem der vorhergehenden Ansprüche, wobei die ionische Flüssigkeit weniger als 5 Gew.-% Halogenid, bevorzugt weniger als 2,5 Gew.-% Halogenid und am bevorzugtesten weniger als 1 Gew.-% Halogenid, umfasst, bevorzugt
wobei die ionische Flüssigkeit weniger als 0,5 Gew.-% Halogenid und bevorzugt weniger als 0,1 Gew.-% Halogenid umfasst, und bevorzugter
wobei die ionische Flüssigkeit frei von Halogenid ist.

12. Schmiermittelzusammensetzung nach einem der vorhergehenden Ansprüche, wobei die Zusammensetzung die eine oder die mehreren ionischen Flüssigkeiten in einer Menge von 0,05 Gew.-% bis 20 Gew.-%, bevorzugt von 0,1 Gew.-% bis 10 Gew.-% und am bevorzugtesten von 0,1 Gew.-% bis 5 Gew.-% umfasst, wie etwa
wobei die Zusammensetzung die eine oder die mehreren ionischen Flüssigkeiten in einer Menge von 0,5 Gew.-% bis 1,5 Gew.-% umfasst.

13. Schmiermittelzusammensetzung nach einem der vorhergehenden Ansprüche, wobei das eine oder die mehreren Basisöle, bezogen auf das Gewicht der Schmiermittelzusammensetzung, in einer Menge von 75% bis 99% in der Zusammensetzung vorhanden sind.

14. Schmiermittelzusammensetzung nach einem der vorhergehenden Ansprüche, wobei das eine oder die mehreren Basisöle einen oder mehrere Basismaterialien ausgewählt aus Gruppe-I-Basismaterialien, Gruppe-II-Basismaterialien, Gruppe-III-Basismaterialien, Gruppe-IV-Basismaterialien und Gruppe-V-Basismaterialien, umfassen, und/oder
wobei das eine oder die mehreren Basisöle ein oder mehrere synthetische Öle, ausgewählt aus Poly-Alpha-Olefinen, synthetischen Estern, Polyalkylenglycolen, Phosphatestern, alkylierten Naphthalenen, Silicatestern, ionischen Fluiden oder mehrfach alkylierten Cyclopentanen, umfassen.

15. Schmiermittelzusammensetzung nach einem der vorhergehenden Ansprüche, die ferner ein oder mehrere Additive umfasst, die optional aus einem oder mehreren Stockpunktadditiven, Antischaumadditiven, Viskositätsindexverbesserern, Antioxidationsmitteln, Detergenzien, Korrosionsinhibitoren, Verschleißminderungsadditiven, Reibwertveränderern, Hochdruckadditiven oder einer beliebigen Kombination davon ausgewählt sind.

16. Schmiermittelzusammensetzung nach einem der vorhergehenden Ansprüche, wobei die Zusammensetzung eine Schmierölzusammensetzung ist, wie etwa
wobei die Zusammensetzung eine Schmierfettzusammensetzung ist; wobei die Schmierfettzusammensetzung optional zu 50 bis 95 Gewichts-% ein oder mehrere Basisöle; und zu 5 bis 50 Gewichts-% ein oder mehrere Verdickungsmittel umfasst; wobei die Schmierfettzusammensetzung bevorzugt zu 10 bis 30 Gewichts-% einen oder mehrere Verdickungsmittel umfasst, bevorzugt
wobei das eine oder die mehreren Verdickungsmittel Seife wie etwa ein Alkalimetall- oder Erdalkalimetallsalz einer C₁₄- bis C₂₀-Fettsäure oder Derivate davon; ein Erdalkalimetallsulfonat wie etwa Calciumsulfonat; einen Harnstoff; einen substituierten Harnstoff; einen Polyharnstoff; einen substituierten Polyharnstoff; Ton; Teer; Graphit; Glimmer; Siliciumdioxid; oder eine Kombination davon umfassen.

17. Ein Verfahren zur Herstellung einer Schmiermittelzusammensetzung nach einem der vorhergehenden Ansprüche, das die Schritte des Vereinigens eines oder mehrerer Basisöle mit einer oder mehreren ionischen Flüssigkeiten und optional einem oder mehreren Additiven oder einem oder mehreren Verdickungsmitteln umfasst.

18. Ein Verfahren zum Betrieb von Industriemaschinen, eines Ottomotors oder eines Verbrennungsmotors mit Selbstzündung, das das Schmieren des genannten Motors mit der Schmiermittelzusammensetzung nach einem der vorhergehenden Ansprüche umfasst.

19. Eine ionische Flüssigkeit, die ein oder mehrere Kationen und ein oder mehrere zweikernige molybdänhaltige Metallatanionen umfasst, wobei die eine oder die mehreren zweikernigen molybdänhaltigen Metallatanionen die Formel [Mo₂A_{b}C_{d}] aufweisen und eine Ladung von -1 oder -2 haben, wobei A und C jeweils unabhängig aus O und S ausgewählt sind; und wobei b und d jeweils 0 bis 8 sind; wobei die Summe von b und d 8 ist, bevorzugt
wobei die ionische Flüssigkeit wie in einem oder mehreren der Ansprüche 2 bis 11 definiert ist.

20. Verwendung einer ionischen Flüssigkeit nach Anspruch 19 in einer Schmiermittelzusammensetzung zu dem Zweck der Verbesserung der reibungsmindernden Eigenschaften der Schmiermittelzusammensetzung und/oder zu dem Zweck der Verbesserung der verschleißmindernden Eigenschaften der Schmiermittelzusammensetzung, bevorzugt
wobei die Schmiermittelzusammensetzung wie in einem der Ansprüche 1 bis 16 definiert ist, bevorzugter
wobei die ionische Flüssigkeit zu dem Zweck der Verbesserung der reibungsmindernden Eigenschaften der Schmiermittelzusammensetzung verwendet wird oder
wobei die ionische Flüssigkeit zu dem Zweck der Verbesserung der verschleißmindernden Eigenschaften der Schmiermittelzusammensetzung verwendet wird.

21. Ein Additivkonzentrat zur Verwendung in einer Schmiermittelzusammensetzung, wobei das Additivkonzentrat eine ionische Flüssigkeit nach Anspruch 19 oder wie in einem der Ansprüche 1 bis 11 definiert und ein oder mehrere weitere Additive, ausgewählt aus einem oder mehreren Stockpunktadditiven, Antischaumadditiven, Viskositätsindexverbesserern, Antioxidationsmitteln, Detergenzien, Korrosionsinhibitoren, Verschleißminderungsadditiven, Reibwertveränderern, Hochdruckadditiven oder einer beliebigen Kombination davon, umfasst.

22. Ein Prozess zur Herstellung einer ionischen Flüssigkeit nach Anspruch 19, wobei der Prozess Folgendes umfasst:
(a) Bereitstellen einer ionischen Flüssigkeit, die ein oder mehrere Kationen wie in einem der Ansprüche 5 bis 9 definiert und ein oder mehrere Anionen der folgenden Formel umfasst: 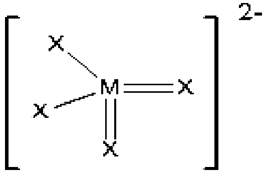 wobei jedes X unabhängig aus O oder S ausgewählt ist; und wobei M = Mo; und
(b) Erwärmen der ionischen Flüssigkeit von Schritt (a) auf eine Temperatur von 70°C bis 130°C in Gegenwart von Luft oder Sauerstoff, um somit die ionische Flüssigkeit nach Anspruch 19 bereitzustellen; wobei die ionische Flüssigkeit bevorzugt auf eine Temperatur von 80°C bis 120°C erwärmt wird, bevorzugter
wobei Schritt (b) ferner das Rühren der ionischen Flüssigkeit von Schritt (a) umfasst und/oder
wobei die eine oder die mehreren Anionen der ionischen Flüssigkeit in Schritt (a) die folgende Formel aufweisen: 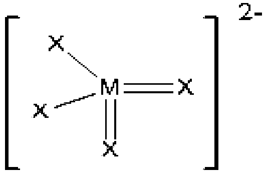 wobei jedes X O ist; oder wobei jedes X S ist; wobei jedes X bevorzugt S ist.

23. Prozess zur Herstellung einer ionischen Flüssigkeit nach Anspruch 19, wobei der Prozess Folgendes umfasst:
(a) Bilden einer Lösung eines Salzes, die ein oder mehrere Kationen und ein oder mehrere Anionen der folgenden Formel umfasst: 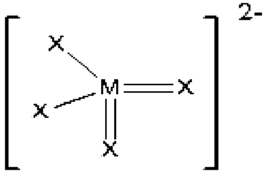 wobei jedes X unabhängig aus O oder S ausgewählt ist; und wobei M = Mo;
(b) Erwärmen der Lösung von Schritt (a) auf eine Temperatur von 70°C bis 130°C in Gegenwart von Luft oder Sauerstoff; wobei die Lösung bevorzugt auf eine Temperatur von 80°C bis 120°C erwärmt wird;
(c) optional Hinzugeben von einer oder mehreren Basen zu der Lösung; und
(d) In-Kontakt-Bringen der Lösung mit einem Salz, das ein oder mehrere Kationen wie in einem der Ansprüche 5 bis 9 definiert und ein oder mehrere Anionen umfasst, um die ionische Flüssigkeit nach Anspruch 19 zu bilden, bevorzugt
wobei Schritt (a) das Rühren des Salzes in einem geeigneten Lösungsmittel umfasst, um die Lösung zu bilden, und/oder
wobei Schritt (d) ferner das Rühren der Lösung nach dem In-Kontakt-Bringen umfasst und/oder
wobei das Salz, das in Schritt (d) der Lösung zugegeben wird, in Form einer Lösung ist.

## Revendications

1. Composition de lubrifiant comprenant une ou plusieurs huiles de base et un ou plusieurs liquides ioniques, dans laquelle les un ou plusieurs liquides ioniques comprennent un ou plusieurs cations et un ou plusieurs anions métallates binucléaires contenant du molybdène, dans laquelle les un ou plusieurs anions métallates binucléaires contenant du molybdène sont de formule [Mo₂A_{b}C_{d}] et ont une charge de -1 ou -2, dans laquelle A et C sont chacun choisis indépendamment parmi O et S ; et dans laquelle b et d valent chacun de 0 à 8 ; dans laquelle la somme de b et d est 8.

2. Composition de lubrifiant selon la revendication 1, dans laquelle les un ou plusieurs anions métallates binucléaires contenant du molybdène ont une charge de -2 ; et/ou dans laquelle chaque atome de molybdène est lié à 5 atomes.

3. Composition de lubrifiant selon l'une quelconque des revendications 1 à 2, dans laquelle les un ou plusieurs anions métallates binucléaires contenant du molybdène sont de formule : 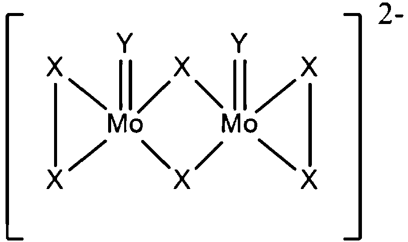 dans laquelle chaque X et chaque Y sont choisis indépendamment parmi O ou S, de préférence
dans laquelle les un ou plusieurs anions métallates binucléaires contenant du molybdène comprennent au moins deux atomes O et/ou au moins deux atomes S, ou
dans laquelle au moins un X est O et au moins un Y est S, ou
dans laquelle au moins un X est S et au moins un Y est O, de préférence
dans laquelle chaque X est S et au moins un Y est O, et plus préférablement
dans laquelle chaque X est S et chaque Y est O.

4. Composition de lubrifiant selon l'une quelconque des revendications précédentes, dans laquelle les un ou plusieurs liquides ioniques ont la formule : 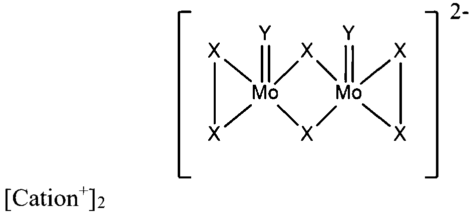 dans laquelle X et Y sont tels que définis dans la revendication 3 ; de préférence dans laquelle chaque X est S et chaque Y est O.

5. Composition de lubrifiant selon l'une quelconque des revendications précédentes, dans laquelle les un ou plusieurs cations comprennent un ou plusieurs cations choisis parmi un cation imidazolium, un cation ammonium quaternaire, un cation phosphonium quaternaire, un cation sulfonium, ou toute combinaison de ceux-ci, de préférence
dans laquelle les un ou plusieurs cations comprennent un ou plusieurs cations choisis parmi les structures suivantes : 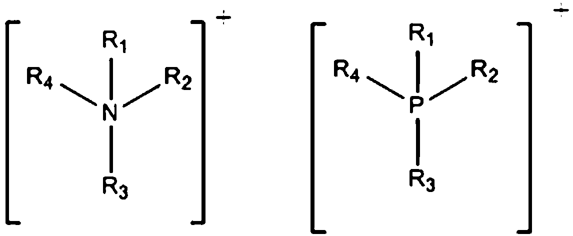 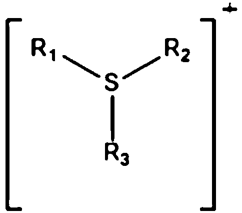 dans laquelle R₁, R₂, R₃ et R₄ sont choisis indépendamment parmi des groupes alkyle et alcényle à chaîne linéaire ou ramifiés en C₁ à C₃₀ ; des groupes cycloalkyle en C₃ à C₆ ; des groupes arylalkyle en C₁ à C₃₀ ; des groupes alkylaryle en C₁ à C₃₀ ; des groupes aryle ; ou deux quelconques de R₁, R₂, R₃ et R₄ se combinent pour former une chaîne alkylène-(CH₂)_{q-} dans laquelle q vaut de 3 à 6 ; dans laquelle un ou plusieurs quelconques desdits groupes alkyle et alcényle à chaîne linéaire ou ramifiée, groupes cycloalkyle, groupes arylalkyle, groupes alkylaryle ; groupes aryle ou chaînes alkylène sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆), plus préférablement
dans laquelle R₁, R₂, R₃ et R₄ sont des groupes alkyle et alcényle à chaîne linéaire ou ramifiés en C₁ à C₃₀, de préférence des groupes alkyle à chaîne linéaire ou ramifiés en C₁ à C₃₀, telle que dans laquelle les un ou plusieurs cations comprennent un ou plusieurs cations choisis parmi les structures suivantes : 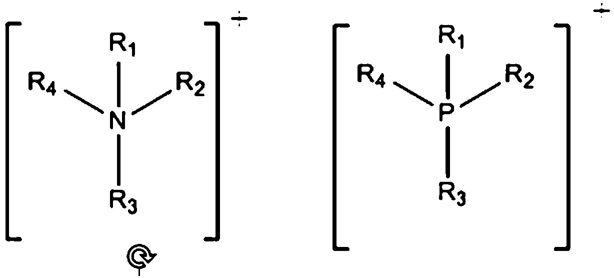 dans laquelle R₁, R₂ et R₃ sont identiques et R₄ est différent de R₁, R₂ et R₃; ou dans laquelle les un ou plusieurs cations comprennent un ou plusieurs cations choisis dans la structure suivante : 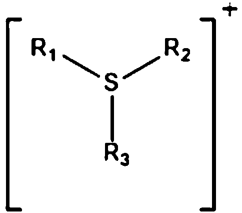 dans laquelle R₁ et R₂ sont identiques et R₃ est différent de R₁ et R₂, de manière encore plus préférée
R₁, R₂, R₃ et R₄ sont des groupes alkyle à chaîne linéaire ou ramifiés en C₁ à C₁₈, de préférence des groupes alkyle à chaîne linéaire en C₁ à C₁₈, et de manière plus préférée entre toutes
dans laquelle les un ou plusieurs cations comprennent un ou plusieurs cations choisis parmi les structures suivantes : 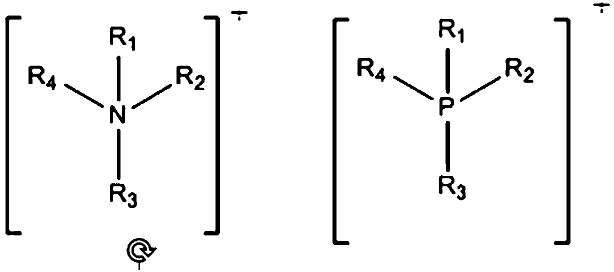 dans laquelle R₁, R₂ et R₃ sont un alkyle à chaîne linéaire en C₄ à C₁₀ et où R₄ est un alkyle à chaîne linéaire en C₁ à C₄, par exemple
dans laquelle R₁, R₂ et R₃ sont un octyle et dans laquelle R₄ est un méthyle, et de préférence
dans laquelle les un ou plusieurs cations comprennent un cation phosphonium.

6. Composition de lubrifiant selon la revendication 5, dans laquelle les un ou plusieurs cations comprennent un ou plusieurs cations choisis parmi les structures suivantes : 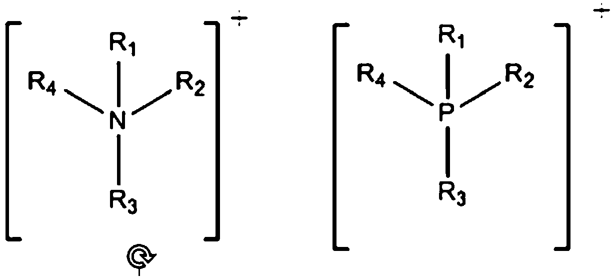 dans laquelle R₁ et R₂ sont un alkyle à chaîne linéaire en C₁₄ à C₂₀ et dans laquelle R₃ et R₄ sont un alkyle à chaîne linéaire en C₁ à C₄ , de préférence
dans laquelle R₁ et R₂ sont un octadécyle et dans laquelle R₃ et R₄ sont un méthyle, et plus préférablement
dans laquelle les un ou plusieurs cations comprennent un cation ammonium.

7. Composition de lubrifiant selon l'une quelconque des revendications précédentes, dans laquelle le liquide ionique comprend du [P_{1,8,8,8}]₂ [Mo₂O₂S₆] ou du [N_{1,1,18,18}] [Mo₂O₂S₆].

8. Composition de lubrifiant selon l'une quelconque des revendications 1 à 5, dans laquelle les un ou plusieurs cations comprennent un cation imidazolium quaternaire substitué comprenant au moins un groupe ester, ou un ou plusieurs cations choisis parmi les structures suivantes : 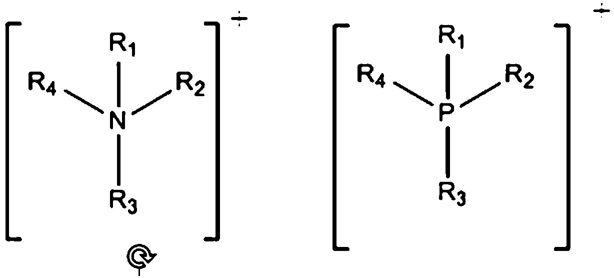 dans laquelle au moins l'un de R₁, R₂, R₃ et R₄ a la formule -R₅O(C=O)R₆ ou -R₅(C=O)-O-R₆, dans laquelle R₅ est un groupe alkyle ou alcényle à chaîne linéaire ou ramifié en C₁ à C₁₀, ou un groupe cycloalkyle ou cycloalcényle en C₃ à C₆ ; et R₆ est un groupe alkyle ou alcényle à chaîne linéaire ou ramifié en C₁ à C₃₀ ; un groupe cycloalkyle en C₃ à C₆ ; un groupe arylalkyle en C₁ à C₃₀ ; un groupe alkylaryle en C₁ à C₃₀ ; un groupe aryle ; ou deux quelconques groupes R₆ respectifs se combinent pour former une chaîne alkylène -(CH₂)q- dans laquelle q vaut de 3 à 6 ; dans laquelle R₅ et/ou R₆ sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ;
et dans laquelle les autres de R₁, R₂, R₃ et R₄ qui ne sont pas tels que définis ci-dessus sont choisis indépendamment parmi des groupes alkyle et alcényle à chaîne linéaire ou ramifiés en C₁ à C₃₀, tels que des groupes alkyle ou alcényle en C₁ à C₁₀ ; des groupes cycloalkyle en C₃ à C₆ ; des groupes arylalkyle en C₁ à C₃₀ ; des groupes alkylaryle en C₁ à C₃₀ ; des groupes aryle ; ou deux quelconques de R₁, R₂, R₃ et R₄ se combinent pour former une chaîne alkylène -(CH₂)q- dans laquelle q vaut de 3 à 6 ; dans laquelle un ou plusieurs quelconques desdits groupes alkyle et alcényle à chaîne linéaire ou ramifiée, groupes cycloalkyle, groupes arylalkyle, groupes alkylaryle ; groupes aryle ou chaînes alkylène sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ; ou dans laquelle les un ou plusieurs cations comprennent un ou plusieurs cations choisis parmi la structure suivante : 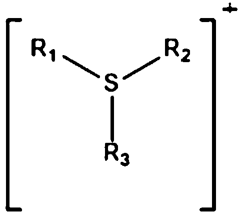 dans laquelle au moins l'un de R₁, R₂, et R₃ a la formule -R₅O(C=O)R₆ ou -R₅(C=O)-O-R₆, dans laquelle R₅ est un groupe alkyle ou alcényle à chaîne linéaire ou ramifié en C₁ à C₁₀, ou un groupe cycloalkyle ou cycloalcényle en C₃ à C₆ ; et R₆ est un groupe alkyle ou alcényle à chaîne linéaire ou ramifié en C₁ à C₃₀ ; un groupe cycloalkyle en C₃ à C₆ ; un groupe arylalkyle en C₁ à C₃₀ ; un groupe alkylaryle en C₁ à C₃₀ ; un groupe aryle ; ou deux quelconques groupes R₆ respectifs se combinent pour former une chaîne alkylène -(CH₂)q- dans laquelle q vaut de 3 à 6 ; dans laquelle R₅ et/ou R₆ sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ;
et dans laquelle les autres des R₁, R₂, et R₃ qui ne sont pas tels que définis ci-dessus sont choisis indépendamment parmi des groupes alkyle et alcényle à chaîne linéaire ou ramifiés en C₁ à C₃₀, tels que des groupes alkyle ou alcényle en C₁ à C₁₀ ; des groupes cycloalkyle en C₃ à C₆ ; des groupes arylalkyle en C₁ à C₃₀ ; des groupes alkylaryle en C₁ à C₃₀ ; des groupes aryle ; ou deux quelconques de R₁, R₂, R₃ et R₄ se combinent pour former une chaîne alkylène -(CH₂)q- dans laquelle q vaut de 3 à 6 ; dans laquelle un ou plusieurs quelconques desdits groupes alkyle et alcényle à chaîne linéaire ou ramifiée, groupes cycloalkyle, groupes arylalkyle, groupes alkylaryle ; groupes aryle ou chaînes alkylène sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆).

9. Composition de lubrifiant selon l'une quelconque des revendications 1 à 5, dans laquelle les un ou plusieurs cations comprennent un cation imidazolium quaternaire substitué comprenant au moins un groupe amide, ou un ou plusieurs cations choisis parmi les structures suivantes : 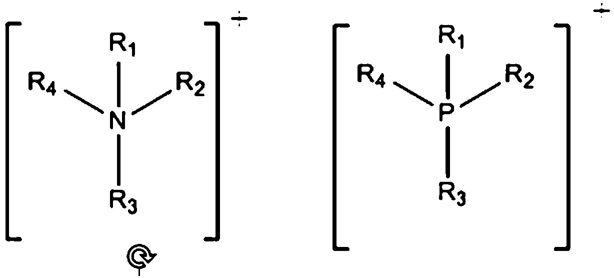 dans laquelle au moins l'un de R₁, R₂, R₃ et R₄ a la formule -R₅-N(R₇)-(C=O)R₆ ou -R₅(C=O)-N(R₇)-R₆, dans laquelle R₅ et R₆ sont tels que définis dans la revendication 23, et dans laquelle R₇ est choisi parmi H ; un groupe alkyle ou alcényle à chaîne linéaire ou ramifié en C₁ à C₃₀ ; un groupe cycloalkyle en C₃ à C₆ ; un groupe arylalkyle en C₁ à C₃₀ ; un groupe alkylaryle en C₁ à C₃₀ ; un groupe aryle ; ou deux quelconques groupes R₆ respectifs se combinent pour former une chaîne alkylène -(CH₂)q- dans laquelle q vaut de 3 à 6 ; dans laquelle R₅ et/ou R₆ sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ;
et dans laquelle les autres de R₁, R₂, R₃ et R₄ qui ne sont pas tels que définis ci-dessus sont choisis indépendamment parmi des groupes alkyle et alcényle à chaîne linéaire ou ramifiés en C₁ à C₃₀, tels que des groupes alkyle ou alcényle en C₁ à C₁₀ ; des groupes cycloalkyle en C₃ à C₆ ; des groupes arylalkyle en C₁ à C₃₀ ; des groupes alkylaryle en C₁ à C₃₀ ; des groupes aryle ; ou deux quelconques de R₁, R₂, R₃ et R₄ se combinent pour former une chaîne alkylène -(CH₂)q- dans laquelle q vaut de 3 à 6 ; dans laquelle un ou plusieurs quelconques desdits groupes alkyle et alcényle à chaîne linéaire ou ramifiée, groupes cycloalkyle, groupes arylalkyle, groupes alkylaryle ; groupes aryle ou chaînes alkylène sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ; ou dans laquelle les un ou plusieurs cations comprennent un ou plusieurs cations choisis parmi la structure suivante : 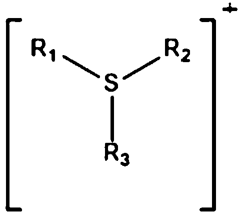 dans laquelle au moins l'un de R₁, R₂, et R₃ a la formule -R₅-N(R₇)-(C=O)R₆ ou -R₅(C=O)-N(R₇)-R₆, dans laquelle R₅ et R₆ sont tels que définis dans la revendication 23, et dans laquelle R₇ est choisi parmi H ; un groupe alkyle ou alcényle à chaîne linéaire ou ramifié en C₁ à C₃₀ ; un groupe cycloalkyle en C₃ à C₆ ; un groupe arylalkyle en C₁ à C₃₀ ; un groupe alkylaryle en C₁ à C₃₀ ; un groupe aryle ; ou deux quelconques groupes R₆ respectifs se combinent pour former une chaîne alkylène -(CH₂)q- dans laquelle q vaut de 3 à 6 ; dans laquelle R₅ et/ou R₆ sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆) ;
et dans laquelle les autres de R₁, R₂, et R₃ qui ne sont pas tels que définis ci-dessus sont choisis indépendamment parmi des groupes alkyle et alcényle à chaîne linéaire ou ramifiés en C₁ à C₃₀, tels que des groupes alkyle ou alcényle en C₁ à C₁₀ ; des groupes cycloalkyle en C₃ à C₆ ; des groupes arylalkyle en C₁ à C₃₀ ; des groupes alkylaryle en C₁ à C₃₀ ; des groupes aryle ; ou deux quelconques de R₁, R₂, R₃ et R₄ se combinent pour former une chaîne alkylène -(CH₂)q- dans laquelle q vaut de 3 à 6 ; dans laquelle un ou plusieurs quelconques desdits groupes alkyle et alcényle à chaîne linéaire ou ramifiée, groupes cycloalkyle, groupes arylalkyle, groupes alkylaryle ; groupes aryle ou chaînes alkylène sont facultativement substitués par un à trois groupes choisis parmi : un alcoxy en C₁ à C₄, un alcoxyalcoxy en C₂ à C₈, un cycloalkyle en C₃ à C₆, -OH, -NH₂, -SH, -CO₂-alkyle en (C₁ à C₆), et -OC(O)-alkyle en (C₁ à C₆), de préférence
dans laquelle les un ou plusieurs cations comprennent un ou plusieurs cations choisis parmi les structures suivantes : 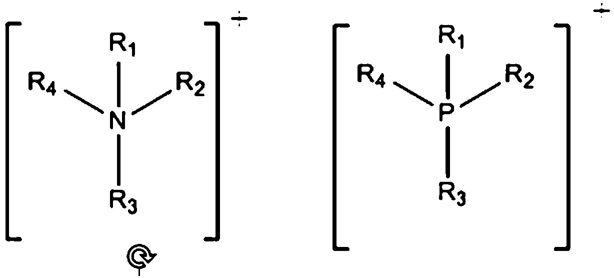 dans laquelle trois des R₁, R₂, R₃, et R₄ ont la formule-R₅O(C=O)R₆ ; -R₅(C=O)-O-R₆ ; -R₅-N(R₇)-(C=O)R₆ ; ou -R₅(C=O)-N(R₇)-R₆ ; dans laquelle R₅, R₆ et R₇ sont tels que définis ci-dessus, plus préférablement
dans laquelle trois des R₁, R₂, R₃, et R₄ ont la formule -R₅O(C=O)R₆, plus préférablement
dans laquelle le cation comprend un cation ammonium quaternaire ou un cation phosphonium quaternaire, tel que
dans laquelle le cation comprend un cation ammonium quaternaire ; R₁ , R₂ et R₃ ont la formule - R₅O(C=O)R₆ dans laquelle R₅ est C₂H₄ ; et dans laquelle R₄ est un alkyle en C₁ à C₅, de préférence un méthyle.

10. Composition de lubrifiant selon l'une quelconque des revendications précédentes, dans laquelle le liquide ionique est un liquide à une température de 100 °C et plus, de préférence 50 °C et plus, et/ou
dans laquelle le liquide ionique est un liquide à une température de 25 °C et plus, de préférence 20 °C et plus, de manière plus préférée 10 °C et plus, et de manière préférée entre toutes 0°C et plus.

11. Composition de lubrifiant selon l'une quelconque des revendications précédentes, dans laquelle le liquide ionique comprend moins de 5 % en poids d'halogénure, de préférence moins de 2,5 % en poids d'halogénure, et de manière préférée entre toutes moins de 1 % en poids d'halogénure, de préférence
dans laquelle le liquide ionique comprend moins de 0,5 % en poids d'halogénure, de préférence moins de 0,1 % en poids d'halogénure, et plus préférablement
dans laquelle le liquide ionique est exempt d'halogénure.

12. Composition de lubrifiant selon l'une quelconque des revendications précédentes, dans laquelle la composition comprend les un ou plusieurs liquides ioniques en une quantité allant de 0,05 % en poids à 20 % en poids, de préférence de 0,1 % en poids à 10 % en poids, et de manière préférée entre toutes de 0,1 % en poids à 5 % en poids, telle que
dans laquelle la composition comprend les un ou plusieurs liquides ioniques en une quantité allant de 0,5 % en poids à 1,5 % en poids.

13. Composition de lubrifiant selon l'une quelconque des revendications précédentes, dans laquelle les une ou plusieurs huiles de base sont présentes dans la composition en une quantité allant de 75 % à 99 % en poids de la composition de lubrifiant.

14. Composition de lubrifiant selon l'une quelconque des revendications précédentes, dans laquelle les une ou plusieurs huiles de base comprennent une ou plusieurs huiles de base choisies parmi les huiles de base du groupe I, les huiles de base du groupe II, les huiles de base du groupe III, les huiles de base du groupe IV et les huiles de base du groupe V, et/ou
dans laquelle les une ou plusieurs huiles de base comprennent une ou plusieurs huiles synthétiques choisies parmi les polyalpha-oléfines, les esters synthétiques, les polyalkylèneglycols, les esters de phosphate, les naphtalènes alkylés, les esters de silicate, les fluides ioniques, ou les cyclopentanes multipliés par alkylation.

15. Composition de lubrifiant selon l'une quelconque des revendications précédentes, comprenant en outre un ou plusieurs additifs facultativement choisis parmi un ou plusieurs additifs de point d'écoulement, additifs anti-mousse, améliorateurs d'indice de viscosité, antioxydants, détergents, inhibiteurs de corrosion, additifs anti-usure, modificateurs de friction, additifs extrême pression, ou toute combinaison de ceux-ci.

16. Composition de lubrifiant selon l'une quelconque des revendications précédentes, dans laquelle la composition est une composition d'huile lubrifiante, telle que
dans laquelle la composition est une composition de graisse ; facultativement, dans laquelle la composition de graisse comprend de 50 % à 95 % en poids d'une ou de plusieurs huiles de base ; et de 5% à 50% en poids d'un ou plusieurs épaississants ; de préférence dans laquelle la composition de graisse comprend de 10 % à 30 % en poids d'un ou plusieurs épaississants, de préférence
dans laquelle les un ou plusieurs épaississants comprennent un savon tel qu'un sel de métal alcalin ou de métal alcalino-terreux d'un acide gras en C₁₄ à C₂₀ , ou des dérivés de celui-ci ; un sulfonate de métal alcalino-terreux tel que le sulfonate de calcium ; une urée ; une urée substituée ; une polyurée ; une polyurée substituée ; de l'argile ; du goudron ; du graphite ; du mica ; de la silice ; ou une combinaison de ceux-ci.

17. Procédé de fabrication d'une composition de lubrifiant selon l'une quelconque des revendications précédentes, comprenant les étapes de combinaison d'une ou de plusieurs huiles de base avec un ou plusieurs liquides ioniques, et facultativement un ou plusieurs additifs ou un ou plusieurs épaississants.

18. Procédé de fonctionnement d'une machine industrielle, d'un moteur à allumage par étincelle ou d'un moteur à allumage par compression comprenant la lubrification dudit moteur avec une composition de lubrifiant selon l'une quelconque des revendications précédentes.

19. Liquide ionique comprenant un ou plusieurs cations et un ou plusieurs anions métallates binucléaires contenant du molybdène, dans lequel les un ou plusieurs anions métallates binucléaires contenant du molybdène sont de formule [Mo₂A_{b}C_{d}] et ont une charge de -1 ou -2, dans lequel A et C sont chacun choisis indépendamment parmi O et S ; et dans lequel b et d vont chacun de 0 à 8 ; dans lequel la somme de b et d est 8, de préférence
dans lequel le liquide ionique est tel que défini dans l'une quelconque des revendications 2 à 11.

20. Utilisation d'un liquide ionique selon la revendication 19, dans une composition de lubrifiant, dans le but d'améliorer les propriétés antifriction de la composition de lubrifiant et/ou
dans le but d'améliorer les propriétés anti-usure de la composition de lubrifiant, de préférence
dans laquelle la composition de lubrifiant est telle que définie dans l'une quelconque des revendications 1 à 16, plus préférablement
dans laquelle le liquide ionique est utilisé dans le but d'améliorer les propriétés antifriction de la composition de lubrifiant, ou
dans laquelle le liquide ionique est utilisé pour améliorer les propriétés antifriction de la composition de lubrifiant.

21. Concentré d'additifs destiné à être utilisé dans une composition de lubrifiant, dans lequel le concentré d'additifs comprend un liquide ionique selon la revendication 19, ou tel que défini dans l'une quelconque des revendications 1 à 11, et un ou plusieurs autres additifs choisis parmi un ou plusieurs additifs de point d'écoulement, additifs anti-mousse, améliorateurs d'indice de viscosité, antioxydants, détergents, inhibiteurs de corrosion, additifs anti-usure, modificateurs de friction, additifs extrême pression, ou toute combinaison de ceux-ci.

22. Processus destiné à fabriquer un liquide ionique selon la revendication 19, dans lequel le processus comprend :
(a) la fourniture d'un liquide ionique comprenant un ou plusieurs cations tels que définis dans l'une quelconque des revendications 5 à 9 et un ou plusieurs anions de la formule suivante : 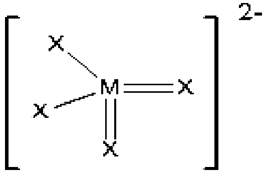 dans lequel chaque X est choisi indépendamment parmi O ou S ; et dans lequel M = Mo ; et
(b) le chauffage du liquide ionique de l'étape (a) à une température allant de 70 °C à 130 °C en présence d'air ou d'oxygène de manière à obtenir le liquide ionique selon la revendication 19 ; de préférence dans lequel le liquide ionique est chauffé à une température allant de 80 °C à 120 °C, plus préférablement
dans lequel l'étape (b) comprend en outre l'agitation du liquide ionique de l'étape (a), et/ou
dans lequel les un ou plusieurs anions du liquide ionique à l'étape (a) sont de la formule suivante : 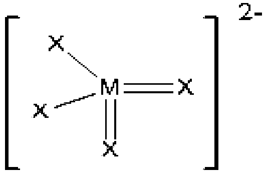 dans lequel chaque X est O ; ou dans lequel chaque X est S ; de préférence dans lequel chaque X est S.

23. Processus destiné à fabriquer un liquide ionique selon la revendication 19, dans lequel le processus comprend :
a) la formation d'une solution d'un sel comprenant un ou plusieurs cations et un ou plusieurs anions de la formule suivante : 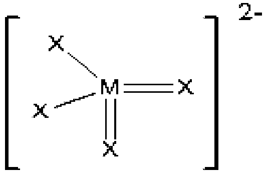 dans lequel chaque X est choisi indépendamment parmi O ou S ; et dans lequel M = Mo ;
(b) le chauffage de la solution de l'étape (a) à une température allant de 70 °C à 130 °C en présence d'air ou d'oxygène ; de préférence dans lequel la solution est chauffée à une température allant de 80 °C à 120 °C ;
(c) facultativement, l'ajout d'une ou de plusieurs bases à la solution ; et
(d) la mise en contact de la solution avec un sel comprenant un ou plusieurs cations tels que définis dans l'une quelconque des revendications 5 à 9 et un ou plusieurs anions pour former le liquide ionique selon la revendication 19, de préférence
dans lequel l'étape (a) comprend l'agitation du sel dans un solvant adapté pour former la solution, et/ou
dans lequel l'étape (d) comprend en outre l'agitation de la solution à la mise en contact, et/ou
dans lequel le sel ajouté à la solution dans l'étape (d) est sous la forme d'une solution.
